# EUROPEAN PATENT APPLICATION

(11) **EP 2 364 716 A2**
(43) Date of publication of application: **14.09.2011**
(21) Application number: 11156084.3
(22) Date of filing: 06.11.2003
(51) Int. Cl.: A61K 38/19, C07K 14/52

(54) **Compositions and methods for the treatment of natural killer cell related diseases**

(30) Priority: 08.11.2002 US 425235 P
(62) Divisional of application: 03786587.0
(71) Applicant: Genentech, Inc., South San Francisco CA 94080-4990 (US)
(72) Inventor: The designation of the inventor has not yet been filed
(74) Representative: Woolley, Lindsey Claire

(57) **Abstract**

The present invention relates to compositions containing novel proteins and methods of using those compositions for the diagnosis and treatment of immune related diseases.

## Description

### Field of the Invention

The present invention relates to compositions and methods useful for the diagnosis and treatment of immune related diseases.

### Background of the Invention

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, *etc*.

Immune related diseases could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

Natural killer (NK) cells are an important effector cell of the innate immune system. They are specialized to effect killing against host cells that have either been infected by viruses, parasites or that have become cancerous. Phenotypically, NK cells are large granular lymphocytes that constitute ∼2 % of the circulating lymphocyte population. They are commonly identified by cell surface expression of CD56 and CD16. NK cells mature in the bone marrow from a CD34+ precursor cell that they share with T cells. The mature NK cell, shares expression of CD8, cytolytic machinery, and some KIRs, with T cells, but remains distinct from T cells by the lack of CD3 and the T cell receptors. Like cytotoxic T cells, they contain granules filled with pore forming protein, cytotoxins, serine esterases and proteoglycans that mediate lysis of target cells. Both cytotoxic T cells and NK cells kill on contact by binding to their targets and delivering their lethal burst of chemicals that produces holes in the target cell's membrane. Unlike cytotoxic T cells, NK cells do not need to recognize a specific antigen before initiating lysis. Rather, NK cell activation can be mediated by growth factors and cytokines such as, IL-2, IL-12 and IL-15 have been shown to mediate proliferative and cytotoxic activities or by a delicate balance between two classes of NK cell receptors, one that activates the cells, and another that inhibits. Killer Ig-like receptors (KIRs) are NK cell receptors that transmit an inhibitory signal if they encounter class I MHC molecules on a cell surface. This is important for killing of both cancerous cells and virally infected cells. Because viruses often suppress class I MHC expression in cells they infect, the virus-infected cell becomes susceptible to killing by NK cells. Likewise, cancer cells have reduced or no class I MHC expression also become susceptible to killing by NK cells. Natural cytotoxicity receptors (NCRs) constitute a family of activating receptors on NK cells. In some effector-target systems, the surface density of NCRs correlates with the cytolytic activity of the NK cells, while in other systems killing requires cooperation between NCR, another activating receptor NKG2D and its adaptor polypeptide DAP10. Additionally, the strength of the stimulatory signals can be influenced by engagement of co-receptors such as 2B4 and NTB-A. The ligands for NCRs and NKG2D, hemoglutanins and MICA, MICB respectively are not expressed by most normal cells, but are induced in most tumor cell lines. Expression of the ligands by tumor cells triggers a dramatic immune response resulting in tumor cell rejection.

Activation of NK cells with IL-15 or IL-12 have been shown to induce both cytotoxic and proliferative effects. Junctional adhesion molecule 2 (JAM2) has been shown to bind to NK cells and has been hypothesized to play a role in lymphocyte extravasation to sites of inflammation. Therefore, a DNA microarray experiment comparing differential expression of genes from these three modes of activation versus resting NK cells has the potential to reveal novel genes or novel gene associations with NK cell activity. Therapeutic antibodies, peptides or small molecules could be developed to target specific genes revealed by these microarrays for the treatment of immune mediated inflammatory diseases and malignancies.

Despite the above research in NK cells, there is a great need for additional diagnostic and therapeutic agents capable of detecting the presence of NK cell mediated disorders in a mammal and for effectively reducing these disorders. Accordingly, it is an objective of the present invention to identify polypeptides that are differentially expressed in activated NK cells as compared to resting NK cells, and to use those polypeptides, and their encoding nucleic acids, to produce compositions of matter useful in the therapeutic treatment and diagnostic detection of NK cell mediated disorders in mammals.

### Summary of the Invention

### A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (*e.g*., neutralizing antibodies) can be used therapeutically where attenuation of the immune response would be beneficial (*e.g*., inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) increasing infiltration of inflammatory cells into a tissue of a mammal in need thereof, (b) stimulating or enhancing an immune response in a mammal in need thereof, (c) increasing the proliferation of NK cells in a mammal in need thereof in response to an antigen, (d) stimulating the activity of NK cells or (e) increasing the vascular permeability. In a further aspect, when the composition comprises an immune inhibiting molecule, the composition is useful for: (a) decreasing infiltration of inflammatory cells into a tissue of a mammal in need thereof, (b) inhibiting or reducing an immune response in a mammal in need thereof, (c) decreasing the activity of NK cells or (d) decreasing the proliferation of NK cells in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, rheumatoid arthritis, osteoarthritis, juvenile chronic arthritis, spondyloarthropathies, systemic sclerosis, idiopathic inflammatory myopathies, Sjögren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:
(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:
(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:
(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:
(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via *ex vivo* gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of NK cells in a mammal comprising administering to said mammal (a) a PRO polypeptide, (b) an agonist of a PRO polypeptide, or (c) an antagonist of a PRO polypeptide, wherein the activity of NK cells in the mammal is increased.

In a still further embodiment, the invention provides a method of decreasing the activity of NK cells in a mammal comprising administering to said mammal (a) a PRO polypeptide, (b) an agonist of a PRO polypeptide, or (c) an antagonist of a PRO polypeptide, wherein the activity of NK cells in the mammal is decreased.

In a still further embodiment, the invention provides a method of increasing the proliferation of NK cells in a mammal comprising administering to said mammal (a) a PRO polypeptide, (b) an agonist of a PRO polypeptide, or (c) an antagonist of a PRO polypeptide, wherein the proliferation of NK cells in the mammal is increased.

In a still further embodiment, the invention provides a method of decreasing the proliferation of NK cells in a mammal comprising administering to said mammal (a) a PRO polypeptide, (b) an agonist of a PRO polypeptide, or (c) an antagonist of a PRO polypeptide, wherein the proliferation of NK cells in the mammal is decreased.

### B. Additional Embodiments

In other embodiments of the present invention, the invention provides vectors comprising DNA encoding any of the herein described polypeptides. Host cell comprising any such vector are also provided. By way of example, the host cells may be CHO cells, *E. coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

### BRIEF DESCRIPTION OF THE DRAWINGS

In the list of figures for the present application, specific cDNA sequences which are differentially expressed in activated Natural Killer (NK) cells as compared to normal resting NK cells are individually identified with a specific alphanumerical designation. These cDNA sequences are differentially expressed in NK cells that are specifically treated as described in Example 1 below.
For a cDNA sequence in the list of figures, the encoded polypeptide is individually identified with the next consecutive alphanumeric designation.

Sequences identified as Figures 1-1477 correspond to SEQ ID NOS: 1-1477 showingthe nucleic acids of the invention and their encoded PRO polypeptides. The List of Figuresthus gives the figure number and the corresponding DNA or PRO number, in which each Figure number corresponds to the same number SEQ ID NO: in the sequence listing. For example, Figure 1 corresponds to SEQ ID NO: 1 of the sequence listing.

### List of Figures

Figure 1: DNA329899, NP_002785.1, 200039_s_at
Figure 2: PRO69614
Figure 3: DNA304680, HSPCB, 200064-at
Figure 4: PR071106
Figure 5: DNA88643, NP_000190.1, 35626_at
Figure 6: PR02455
Figure 7: DNA331286, NP_006143.1, 35974_at
Figure 8: PRO86388
Figure 9: DNA331287, NP_036455.1, 36711_at
Figure 10: PR083282
Figure 11: DNA254127, NP_008925.1, 38241_at
Figure 12: PR049242
Figure 13A-B: DNA103216, BAA31595.1, 38671_at
Figure 14: PR04546
Figure 15A-B: DNA329093, NP_006631.1, 41220_at
Figure 16: PR084745
Figure 17: DNA326185, NP_073607.2, 45633_at
Figure 18: PRO82602
Figure 19: DNA329913, SSB-3, 46256_at
Figure 20: PRO85228

Figure 21: DNA331288, ARHGAP8, 47069_at
Figure 22: PRO86389
Figure 23: DNA328364, NP_068577.1, 52940_at
Figure 24: PR084223
Figure 25: DNA324633, BC000478, 200691_s_at
Figure 26: PRO81277
Figure 27: DNA324633, NP_004125.2, 200692_s_at
Figure 28: PRO81277
Figure 29: DNA88350, NP_000168.1, 200696_s_at
Figure 30: PR02758
Figure 31: DNA287207, NP_006316.1, 200750_s_at
Figure 32: PR039268
Figure 33: DNA324135, BC001854, 200768_s_at
Figure 34: PRO80837
Figure 35: DNA324135, NP_005902.1, 200769_s_at
Figure 36: PRO80837
Figure 37: DNA324060, NP_002530.1, 200790_at
Figure 38: PR080773
Figure 39: DNA287211, NP_002147.1, 200806_s_at
Figure 40: PR069492

Figure 41: DNA287211, HSPD1, 200807_s_at
Figure 42: PR069492
Figure 43A-B: DNA255281, NP_006380.1, 200825_s_at
Figure 44: PR050357
Figure 45: DNA328380, HSHLAEHCM, 200904_at
Figure 46: DNA272695, NP_001722.1, 200920_s_at
Figure 47: PRO60817
Figure 48: DNA327255, NP_002385.2, 200924_s_at
Figure 49: PR057298
Figure 50: DNA225878, NP_004334.1, 200935-at
Figure 51: PR036341
Figure 52A-B: DNA287217, NP_001750.1, 200951_s_at
Figure 53: PR036766
Figure 54A-B: DNA287217, CCND2, 200952_s_at
Figure 55: PRO36766
Figure 56A-B: DNA226303, HUMRSC289, 200953_s_at
Figure 57: PRO36766
Figure 58A-B: DNA331289, ABLIM1, 200965_s_at
Figure 59: PRO86390
Figure 60: DNA328388, BC010273, 201013_s_at

Figure 61: PR084240
Figure 62: DNA328388, NP_006443.1, 201014_s_at
Figure 63: PR084240
Figure 64: DNA328391, NP_004408.1, 201041_s_at
Figure 65: PR084242
Figure 66: DNA287198, NP_006073.1, 201090_x_at
Figure 67: PR069484
Figure 68: DNA304719, NP_002296.1, 201105_at
Figure 69: PR071145
Figure 70: DNA329931, AF053642, 201111_at
Figure 71: DNA273865, NP_006221.1, 201115_at
Figure 72: PR061824
Figure 73: DNA326273, NP_001961.1, 201123_s_at
Figure 74: PR082678
Figure 75: DNA329103, NP_002112.2, 201137_s_at
Figure 76: PR084752
Figure 77: DNA329104, NP_004085.1, 201144_s_at
Figure 78: PRO69550
Figure 79: DNA151802, NP_003661.1, 201169_s_at
Figure 80: PRO12890

Figure 81: DNA151802, BHLHB2, 201170_s_at
Figure 82: PRO12890
Figure 83A-B: DNA103453, HUME16GEN, 201195_s_at
Figure 84: PRO4780
Figure 85: DNA103488, NP_002583.1, 201202_at
Figure 86: PRO4815
Figure 87: DNA287173, ENO1, 201231_s_at
Figure 88: PRO69463
Figure 89: DNA287331, NP_002645.1, 201251_at
Figure 90: PR069595
Figure 91: DNA270950, NP_003182.1, 201263_at
Figure 92: PRO59281
Figure 93: DNA328405, NP_112556.1, 201277_s_at
Figure 94: PR084252
Figure 95: DNA328406, NP_001334.1, 201279_s_at
Figure 96: PRO84253
Figure 97: DNA328406, DAB2, 201280_s_at
Figure 98: PR084253
Figure 99: DNA331290, NP_038474.1, 201285_at
Figure 100: PRO86391

Figure 101: DNA327546, HSTOP2A10, 201292_at
Figure 102: DNA329106, NP_003013.1, 201311_s_at
Figure 103: PR083360
Figure 104: DNA329002, NP_001753.1, 201327_s_at Figure 105: PRO4912
Figure 106A-B: DNA274141, AF205218, 201362_at
Figure 107: PR062077
Figure 108A-B: DNA331291, AB020657, 201363_s_at
Figure 109: PR062077
Figure 110: DNA329107, NP_008818.3, 201367_s_at
Figure 111: PRO84754
Figure 112A-B: DNA329108, 1383643.16, 201368_at
Figure 113: PR084755
Figure 114: DNA329107, ZFP36L2, 201369_s_at
Figure 115: PR084754
Figure 116: DNA329939,1393503.1, 201417_at
Figure 117: PRO85248
Figure 118: DNA226600, NP_003371.1, 201426_s_at
Figure 119: PR037063
Figure 120: DNA272286, NP_001743.1, 201432_at

Figure 121: PR060544
Figure 122: DNA325704, NP_004981.2, 201475_x_at
Figure 123: PR082188
Figure 124: DNA327551, NP_001024.1, 201477_s_at
Figure 125: PR059289
Figure 126: DNA304459, BC005020, 201489_at
Figure 127: PR037073
Figure 128: DNA304459, NP_005720.1, 201490_s_at
Figure 129: PR037073
Figure 130: DNA323741, NP_003123.1, 201516_at
Figure 131: PRO80498
Figure 132: DNA331292, NP_002779.1, 201532-at
Figure 133: PR084262
Figure 134: DNA272171, NP_002379.2, 201555at
Figure 135: PR060438
Figure 136: DNA227071, NP_000260.1, 201577.at
Figure 137: PR037534
Figure 138A-B: DNA329944, AB032988, 201581.at
Figure 139: DNA272904, NP_006784.1, 201619_at
Figure 140: PR060991

Figure 141: DNA329115, NP-434702.1, 201631_s_at
Figure 142: PRO84760
Figure 143A-B: DNA331293, AB011155, 201681_s_at
Figure 144: DNA270883, NP_001061.1, 201714_at
Figure 145: PR059218
Figure 146: DNA327559, NP_058432.1, 201752_s_at
Figure 147: PR083589
Figure 148: DNA331294, ADD3, 201753_s_at
Figure 149: PR086393
Figure 150: DNA227035, NP_006730.1, 201755_at
Figure 151: PR037498
Figure 152: DNA287167, NP_006627.1, 201761_at
Figure 153: PRO59136
Figure 154: DNA329952, NET1, 201830_s_at
Figure 155: PRO85256
Figure 156: DNA329118, NP_068660.1, 201853_s_at
Figure 157: PR083123
Figure 158A-B: DNA331295, NP_002710.1, 201877_s_at
Figure 159: PR086394
Figure 160: DNA150805, NP_055703.1, 201889_at

Figure 161: PRO11583
Figure 162: DNA253582, DNA253582, 201890_at
Figure 163: PRO49181
Figure 164: DNA329956, NP_000875.1, 201892_s_at
Figure 165: PR085260
Figure 166: DNA328431, NP_001817.1, 201897_s_at
Figure 167: PR045093
Figure 168: DNA227112, NP_006397.1, 201923_at
Figure 169: PR037575
Figure 170: DNA275240, NP_005906.2, 201930_at
Figure 171: PR062927
Figure 172: DNA274167, NP_006422.1, 201946_s_at
Figure 173: PR062097
Figure 174: DNA275214, NP_002473.1, 201970_s_at
Figure 175: PR062908
Figure 176: DNA88666, NP_002334.1, 202018_s_at
Figure 177: PR02892
Figure 178A-B: DNA225991, NP_000518.1, 202068_s_at
Figure 179: PR036454
Figure 180: DNA327567, NP_005521.1, 202069_s_at

Figure 181: PR083596
Figure 182A-B: DNA269686, NP_002994.1, 202083_s_at
Figure 183: PRO58096
Figure 184: DNA328440, NP_004517.1, 202107_s_at
Figure 185: PR084274
Figure 186A-B: DNA304990, NP_006394.1, 202150_s_at
Figure 187: PRO71288
Figure 188: DNA328442, NP_006078.2, 202154_x_at
Figure 189: PR084275
Figure 190: DNA328444, MGC14458, 202246_s_at
Figure 191: PR084277
Figure 192: DNA304716, NP_510867.1, 202284_s_at
Figure 193: PRO71142
Figure 194: DNA270142, NP_005947.2, 202309_at
Figure 195: PR058531
Figure 196: DNA328448, NP_000777.1, 202314_at
Figure 197: PR062362
Figure 198: DNA331296, UNG, 202330_s_at
Figure 199: PR086395
Figure 200: DNA325115, NP_001435.1, 202345_s_at

Figure 201: PR081689
Figure 202: DNA331297, MXI1, 202364_at
Figure 203: PR086396
Figure 204A-B: DNA150955, NP_036420.1, 202369_s_at
Figure 205: PRO12559
Figure 206: DNA290234, NP_002914.1, 202388_at
Figure 207: PR070333
Figure 208: DNA66487, NP_002458.1, 202431_s_at
Figure 209: PRO1213
Figure 210: DNA325532, NP_009104.1, 202458_at
Figure 211: PR0307
Figure 212A-B: DNA269642, NP_004557.1, 202464_s_at
Figure 213: PR058054
Figure 214: DNA227921, NP_003789.1, 202468_s_at
Figure 215: PR038384
Figure 216: DNA329123, NP_002873.1, 202483_s_at
Figure 217: PRO84765
Figure 218A-B: DNA103449, NP_008862.1, 202497_x_at
Figure 219: PR04776
Figure 220A-B: DNA103449, SLC2A3, 202499_s_at

Figure 221: PR04776
Figure 222: DNA234442, NP_055551.1, 202503_s_at
Figure 223: PR038852
Figure 224A-B: DNA277809, NP_055582.1, 202524_s_at
Figure 225: PR064556
Figure 226A-B: DNA255105, NP_000850.1, 202539_s_at
Figure 227: PR050187
Figure 228A-B: DNA255105, HMGCR, 202540_s_at
Figure 229: PR050187
Figure 230: DNA275244, DNA275244, 202557_at
Figure 231: DNA329979, NP_001062.1, 202589_at
Figure 232: PR082821
Figure 233: DNA274881, NP_001896.1, 202613_at
Figure 234: PRO62626
Figure 235: DNA59763, NP-000192.1, 202637_s_at
Figure 236: PRO160
Figure 237: DNA59763, ICAM1, 202638_s_at
Figure 238: PR0160
Figure 239: DNA254129, NP_006001.1, 202655_at
Figure 240: PRO49244

Figure 241: DNA326896, NP_003672.1, 202671_s_at
Figure 242: PR069486
Figure 243: DNA274690, NP_008869.1, 202690_s_at
Figure 244: PR062479
Figure 245: DNA331298, NP_055271.2, 202730_s_at
Figure 246: PRO81909
Figure 247: DNA331299, PDCD4, 202731_at
Figure 248: PR086397
Figure 249: DNA150713, NP_006570.1, 202735_at
Figure 250: PR012082
Figure 251A-B: DNA328464, 977954.20, 202769_at
Figure 252: PR084290
Figure 253: DNA226578, NP_004345.1, 202770_s_at
Figure 254: PR037041
Figure 255: DNA273346, NP_055316.1, 202779_s_at
Figure 256: PR061349
Figure 257A-B: DNA226364, NP_001612.1, 202820_s_at
Figure 258: PR036827
Figure 259: DNA329988, NP_036460.1, 202843_at
Figure 260: PRO1471

Figure 261: DNA328483, NP_061163.1, 202911_at
Figure 262: PR084309
Figure 263: DNA331300, BIN1, 202931_x_at
Figure 264: PRO86398
Figure 265A-B: DNA331301, NP_005424.1, 202932_at
Figure 266: PRO12922
Figure 267A-B: DNA331302, YES1, 202933_s_at
Figure 268: PRO1292
Figure 269: DNA329134, BC012085, 202951_at
Figure 270: PR059254
Figure 271A-B: DNA328473, NP_006473.1, 202968_s_at
Figure 272: PRO84299
Figure 273A-B: DNA194837, NP_055714.1, 202975_s_at
Figure 274: PR024100
Figure 275A-B: DNA194837, RHOBTB3, 202976_s_at
Figure 276: PR024100
Figure 277: DNA329136, NP_057475.1, 203023_at
Figure 278: PR084772
Figure 279A-B: DNA271865, NP_055566.1, 203037_s_at
Figure 280: PR060145

Figure 281: DNA269918, NP_003633.1, 203138_at
Figure 282: PRO58316
Figure 283A-B: DNA331303, NP_003129.1, 203181_s_at
Figure 284: PR086399
Figure 285: DNA331304, BC015747, 203221_at
Figure 286: PR084167
Figure 287: DNA328294, NP_005068.2, 203222_s_at
Figure 288: PR084167
Figure 289A-C: DNA274481, NP_000323.1, 203231_s_at
Figure 290: PR062384
Figure 291A-C: DNA274481, SCA1, 203232_s_at
Figure 292: PR062384
Figure 293: DNA76514, NP_000409.1, 203233_at
Figure 294: PR02540
Figure 295: DNA327590, NP_003355.1, 203234_at
Figure 296: PR083608
Figure 297: DNA325507, NP_005842.1, 203252_at
Figure 298: PR069461
Figure 299: DNA302020, NP_005564.1, 203276_at
Figure 300: PR070993

Figure 301A-B: DNA331305, NP_055716.1, 203286_at
Figure 302: PR086400
Figure 303: DNA271959, NP_002885.1, 203344_s_at
Figure 304: PRO60234
Figure 305: DNA324514, NP_002349.1, 203362_s_at
Figure 306: PRO81169
Figure 307: DNA325749, NP_003868.1, 203372_s_at
Figure 308: PR012839
Figure 309: DNA325749, STATI2, 203373_at
Figure 310: PRO12839
Figure 311: DNA329140, NP_476433.1, 203391_at
Figure 312: PRO84775
Figure 313: DNA323927, NP_005563.1, 203411_s_at
Figure 314: PR080660
Figure 315: DNA151037, NP_036461.1, 203414_at
Figure 316: PRO1258
Figure 317A-B: DNA256807, NP_057339.1, 203420_at
Figure 318: PRO51738
Figure 319A-B: DNA275186, DNA275186, 203432_at
Figure 320A-B: DNA330010, NP_005721.2, 203445_s_at

Figure 321: PRO85299
Figure 322: DNA331306, NP_001715.1, 203502_at
Figure 323: PR058024
Figure 324A-B: DNA331307, NP_003096.1, 203509_at
Figure 325: PRO86401
Figure 326A-B: DNA272399, NP_001197.1, 203542_s_at
Figure 327: PRO60653
Figure 328A-B: DNA272399, BTEB1, 203543_s_at
Figure 329: PR060653
Figure 330: DNA287246, NP_004044.2, 203612_at
Figure 331: PRO69521
Figure 332A-B: DNA331308, BCL2, 203685_at
Figure 333: PRO86402
Figure 334: DNA324183, DPP4, 203716_s_at
Figure 335: PRO80881
Figure 336: DNA196562, HSPCHDP7, 203717_at
Figure 337: PR025042
Figure 338: DNA150784, NP_001974.1, 203720_s_at
Figure 339: PRO12800
Figure 340A-B: DNA325369, NP_055877.2, 203737_s_at
Figure 341: PRO81905
Figure 342: DNA275339, NP_005685.1, 203880_at
Figure 343: PRO63012
Figure 344: DNA82376, NP_002407.1, 203915_at
Figure 345: PRO1723
Figure 346: DNA272338, NP_001245.1, 203967_at
Figure 347: PR060595
Figure 348: DNA272338, CDC6, 203968_s_at
Figure 349: PR060595
Figure 350: DNA271374, NP_005474.1, 203976_s_at
Figure 351: PR059673
Figure 352: DNA329145, DUSP4, 204014_at
Figure 353: PRO84780
Figure 354: DNA330034, NP_002907.1, 204023_at
Figure 355: PRO85319
Figure 356: DNA328271, NP_008988.2, 204026_s_at
Figure 357: PRO81868
Figure 358: DNA226737, NP_004576.1, 204070_at
Figure 359: PR037200
Figure 360A-C: DNA331309, NP_075463.1, 204072_s_at

Figure 361: PR086403
Figure 362: DNA216689, NP_002975.1, 204103_at
Figure 363: PR034276
Figure 364: DNA304489, NP_003495.1, 204126_s_at
Figure 365: PR071058
Figure 366: DNA330037, BC000149, 204127_at
Figure 367: PRO82290
Figure 368: DNA325824, NP_002906.1, 204128_s_at
Figure 369: PRO82290
Figure 370: DNA272655, NP_001818.1, 204170_s_at
Figure 371: PRO60781
Figure 372: DNA226881, NP_002008.2, 204236_at
Figure 373: PR037344
Figure 374A-B: DNA287273, NP_006435.1, 204240_s_at
Figure 375: PR069545
Figure 376: DNA228132, NP_076995.1, 204256_at
Figure 377: PR038595
Figure 378: DNA273802, NP_066950.1, 204285_s_at
Figure 379: PRO61763
Figure 380: DNA273802, PMAIP1, 204286_s_at

Figure 381: PRO61763
Figure 382: DNA331310, NP_000472.1, 204294_at
Figure 383: PRO86404
Figure 384: DNA150972, NP_005252.1, 204472_at
Figure 385: PRO12162
Figure 386A-B: DNA331311, NP_056054.1, 204500_s_at
Figure 387: PRO86405
Figure 388: DNA331312, NP_003600.2, 204504_s_at
Figure 389: PR082595
Figure 390: DNA225993, NP_000646.1, 204563_at
Figure 391: PR036456
Figure 392A-B: DNA330054, NP_004746.1, 204633_s_at
Figure 393: PRO85334
Figure 394: DNA103526, LRMP, 204674_at
Figure 395: PR04853
Figure 396A-B: DNA331313, 481411.2, 204695_at
Figure 397: PRO38036
Figure 398A-B: DNA325192, NP_038203.1, 204744_s_at
Figure 399: PR081753
Figure 400: DNA330060, NP_002443.2, 204766_s_at

Figure 401: PRO85340
Figure 402: DNA329154, BC000323, 204767_s_at
Figure 403: PR069568
Figure 404: DNA325479, NP_004102.1, 204768_s_at
Figure 405: PRO69568
Figure 406: DNA330062, NP_006017.1, 204805_s_at
Figure 407: PR085342
Figure 408: DNA254127, BTN3A3, 204821_at
Figure 409: PR049242
Figure 410: DNA328544, NP_006673.1, 204834_at
Figure 411: PRO84347
Figure 412: DNA328546, NP_005249.1, 204867_at
Figure 413: PR084349
Figure 414: DNA329157, NP_004271.1, 204905_s_at
Figure 415: PRO62861
Figure 416: DNA331095, NP_005216.1, 204947_at
Figure 417: PRO86245
Figure 418: DNA325061, NP_005208.1, 205033_s_at
Figure 419: PR09980
Figure 420: DNA328297, NP_477097.1, 205034_at

Figure 421: PRO59418
Figure 422: DNA324991, ASNS, 205047_s_at
Figure 423: PRO81585
Figure 424: DNA331314, NP_055366.1, 205086_s_at
Figure 425: PRO86406
Figure 426: DNA330074, HUMHM145, 205098_at
Figure 427: PRO85353
Figure 428: DNA226177, NP_001286.1, 205099_s_at
Figure 429: PR036640
Figure 430: DNA192060, NP_002974.1, 205114_s_at
Figure 431: PR021960
Figure 432: DNA299899, NP_002148.1, 205133_s_at
Figure 433: PR062760
Figure 434: DNA273535, NP_004217.1, 205214_at
Figure 435: PR061515
Figure 436: DNA227081, NP_000390.2, 205249_at
Figure 437: PR037544
Figure 438A-B: DNA188301, NP_002300.1, 205266_at
Figure 439: PR021834
Figure 440A-B: DNA331315, LRP8, 205282_at
Figure 441: PR070090
Figure 442: DNA227173, NP_001456.1, 205285_s_at
Figure 443: PRO37636
Figure 444A-B: DNA331316, 983055.1, 205296_at
Figure 445: PR086407
Figure 446: DNA325568, NP.001265.1, 205393_s_at
Figure 447: PR012187
Figure 448: DNA325568, CHEK1, 205394_at
Figure 449: PR012187
Figure 450: DNA328566, NP_060446.1, 205510_s_at
Figure 451: PR084363
Figure 452: DNA330085, D86324, 205518_s_at
Figure 453: PRO85359
Figure 454: DNA330086, NP_079184.1, 205519_at
Figure 455: PR085360
Figure 456: DNA254810, NP_056536.1, 205527_s_at
Figure 457: PR049906
Figure 458: DNA328567, NP_006797.2, 205548_s_at
Figure 459: PRO84364
Figure 460
: DNA329013, NP_005649.1, 205599_at
Figure 461: PR020128
Figure 462: DNA330088, NP_003087.1, 205644_s_at
Figure 463: PR061962
Figure 464: DNA330089, NP_004200.2, 205691_at
Figure 465: PRO12507
Figure 466: DNA331317, NP_003636.1, 205768_s_at
Figure 467: PRO51139
Figure 468: DNA331318, SLC27A2, 205769_at
Figure 469: PR051139
Figure 470: DNA76517, NP_002176.1, 205798_at
Figure 471: PR02541
Figure 472: DNA194766, NP_079504.1, 205804_s_at
Figure 473: PR024046
Figure 474A-B: DNA331319, NP_004749.1,
205839_s_at
Figure 475: PRO86408
Figure 476: DNA327651, NP.005612.1, 205863_at
Figure 477: PRO83649
Figure 478: DNA331320, HSU37122, 205882_x_at
Figure 479: PRO86409
Figure 480: DNA287318, NP_002683.1, 205909_at

Figure 481: PR069583
Figure 482: DNA329166, NP_006518.1, 206052_s_at
Figure 483: PRO84793
Figure 484: DNA329168, CLC, 206207_at
Figure 485: PR084794
Figure 486: DNA281446, NP_031394.1, 206220_s_at
Figure 487: PR066285
Figure 488: DNA331321, NP_057473.1, 206245_s_at
Figure 489: PRO86410
Figure 490A-B: DNA331322, NP_055523.1, 206316_s_at
Figure 491: PRO86411
Figure 492: DNA218278, NP_000408.1, 206341_at
Figure 493: PR034330
Figure 494: DNA329169, NP_002986.1, 206366_x_at
Figure 495: PRO1610
Figure 496A-B: DNA225567, NP_004659.1, 206522_at
Figure 497: PR036030
Figure 498: DNA227540, NP_003036.1, 206566-at
Figure 499: PR038003

Figure 500: DNA329171, NP_060246.1, 206583_at
Figure 501: PRO84795
Figure 502: DNA88374, NP_002095.1, 206666_at
Figure 503: PR02768
Figure 504: DNA330105, HUMNCAX, 206676_at
Figure 505: PRO85372
Figure 506: DNA328590, C6orf32, 206707_x_at
Figure 507: PR084375
Figure 508: DNA325853, NP_075387.1, 206958_s_at
Figure 509: PRO82315
Figure 510: DNA35629, NP_000586.2, 206975_at
Figure 511: PR07
Figure 512: DNA188346, NP_001450.1, 206980_s_at
Figure 513: PR021766
Figure 514A-B: DNA227659, NP_000570.1, 206991_s_at
Figure 515: PR038122
Figure 516A-B: DNA227750, NP_001550.1, 206999_at
Figure 517: PRO38213
Figure 518: DNA188289, NP_001548.1, 207008_at
Figure 519: PR021820
Figure 520: DNA255987, NP_004685.1, 207038_at

Figure 521: PRO51038
Figure 522: DNA227481, VAMP1, 207100_s_at
Figure 523: PR037944
Figure 524: DNA218655, NP_000585.1, 207113_s_at
Figure 525: PRO34451
Figure 526A-B: DNA327674, NP_002739.1, 207121_s_at
Figure 527: PRO83661
Figure 528: DNA331323, NP_001250.1, 207143_at
Figure 529: PRO86412
Figure 530: DNA83048, NP_001916.1, 207269_at
Figure 531: PR02057
Figure 532: DNA331324, LTB, 207339_s_at
Figure 533: PRO11604
Figure 534: DNA226396, NP_002180.1, 207375_s_at
Figure 535: PR036859
Figure 536: DNA329178, BTN3A1, 207485_x_at
Figure 537: PRO84801
Figure 538: DNA304473, NP_001552.2, 207536_s_at
Figure 539: PR02023
Figure 540A-B: DNA330120, FLJ10971, 207606_s_at

Figure 541: PR085382
Figure 542: DNA227606, NP_001872.2, 207630_s_at
Figure 543: PR038069
Figure 544: DNA196426, NP_037440.1, 207651_at
Figure 545: PRO24924

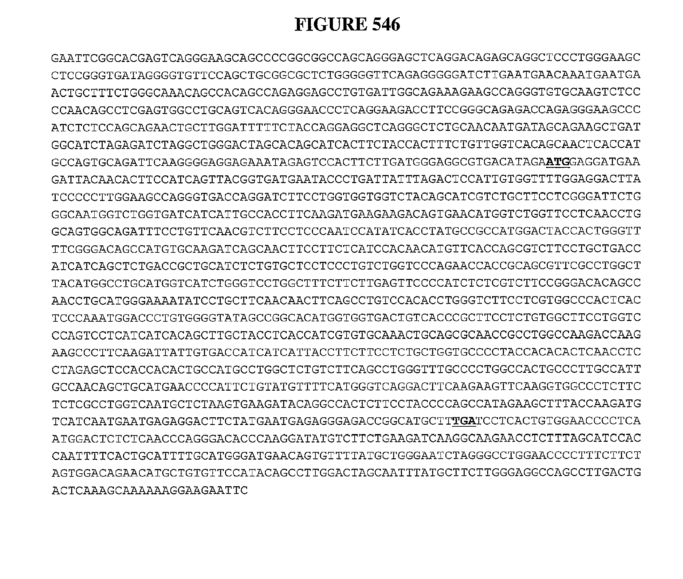

Figure 546: DNA256401, NP_004063.1, 207652_s_at

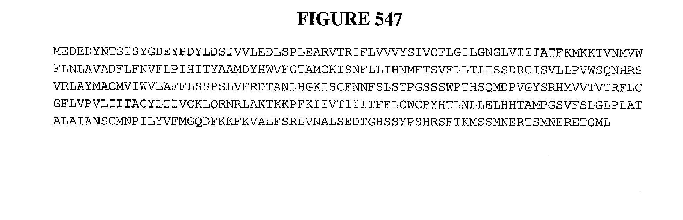

Figure 547: PRO51442,
Figure 548: DNA328763, NP-001219.2, 207686_s_at
Figure 549: PRO84511
Figure 550: DNA325654, NP_054752.1, 207761_s_at
Figure 551: PR04348
Figure 552: DNA329184, CITED2, 207980_s_at
Figure 553: PRO84807
Figure 554: DNA227224, NP_060877.1, 208029_s_at
Figure 555: PR037687
Figure 556: DNA328610, NP-112601.2, 208146_at
Figure 557: PR084392
Figure 558: DNA325329, NP_004719.1, 208152_s_at
Figure 559: PRO81872
Figure 560: DNA328611, RASGRP2, 208206_s_at
Figure 561: PR084393
Figure 562: DNA331325, BC006443, 208433_s_at
Figure 563: DNA103427, NP-005239.1, 208438_s_at
Figure 564: PRO4755
Figure 565A-C: DNA331326, ATM, 208442_s_at
Figure 566: PRO86413
Figure 567: DNA331327, NP_036382.2, 208456_s_at
Figure 568: PRO86414
Figure 569: DNA331328, NP_000690.1, 208498_s_at
Figure 570: PR02157
Figure 571A-B: DNA273567, NP_004944.1, 208625_s_at
Figure 572: PR061545
Figure 573: DNA329188, BC012142, 208638_at
Figure 574: PRO84810
Figure 575: DNA330139, AK022493, 208657_s_at
Figure 576: PRO85398
Figure 577: DNA304686, NP_002565.1, 208680_at
Figure 578: PR071112
Figure 579: DNA287189, NP_002038.1, 208693_s_at
Figure 580: PRO69475

Figure 581: DNA330140, AF275798, 208696_at
Figure 582: PRO85399
Figure 583: DNA327696, AF228339, 208763_s_at
Figure 584: PRO83679
Figure 585: DNA238565, NP_005907.2, 208795_s_at
Figure 586: PR039210
Figure 587: DNA330145, NP_002788.1, 208799_at
Figure 588: PR084403
Figure 589: DNA273521, NP_002070.1, 208813_at
Figure 590: PR061502
Figure 591: DNA227874, NP_003320.1, 208864_s_at
Figure 592: PR038337
Figure 593: DNA328624, BC003562, 208891_at
Figure 594: PR059076
Figure 595: DNA331329, DUSP6, 208892_s_at
Figure 596: PRO84404
Figure 597: DNA331330, BC005047, 208893_s_at
Figure 598: PRO82215
Figure 599: DNA327701, NP_001203.1, 208910_s_at
Figure 600: PR082667
Figure 601: DNA226500, NP_005619.1, 208916_at
Figure 602: PR036963
Figure 603: DNA329552, NP_063948.1, 208925_at
Figure 604: PRO85097
Figure 605: DNA328629, NP_006079.1, 208977_x_at
Figure 606: PRO84407
Figure 607: DNA330154, HUMPECAM27, 208981_at
Figure 608: DNA330155, 7692317.2, 208982_at
Figure 609: PR085407
Figure 610: DNA328631, AK027318, 209006_s_at
Figure 611: PRO84409
Figure 612: DNA328632, DJ465N24.2.1Homo, 209007_s_at
Figure 613: DNA328635, BC020946, 209026_x_at
Figure 614: PRO84413
Figure 615: DNA274202, NP_006804.1, 209034_at
Figure 616: PRO62131
Figure 617A-C: DNA328637, HSA7042, 209053_s_at
Figure 618: PR081109
Figure 619: DNA327713, BC010653, 209146_at
Figure 620: PR037975

Figure 621A-B: DNA328642, AF073310, 209184_s_at
Figure 622: PRO84418
Figure 623: DNA331331, AF161416, 209185_s_at
Figure 624A-B: DNA328643, HUMHK1A, 209186_at
Figure 625: PRO84419
Figure 626: DNA103520, NP_002639.1, 209193_at
Figure 627: PR04847
Figure 628: DNA226176, NP_003458.1, 209201_x_at
Figure 629: PR036639
Figure 630: DNA326267, NP_004861.1, 209208_at
Figure 631: PRO82674
Figure 632: DNA328645, NP_009006.1, 209216_at
Figure 633: PRO84421
Figure 634: DNA227483, NP_003120.1, 209218_at
Figure 635: PR037946
Figure 636: DNA331332, BC007405, 209238_at
Figure 637: PRO86416
Figure 638A-B: DNA331333, 371440.32, 209240_at
Figure 639: PRO86417
Figure 640: DNA328649, NP_116093.1, 209251_x_at

Figure 641: PRO84424
Figure 642: DNA255255, NP_071437.1, 209267_s_at
Figure 643: PR050332
Figure 644: DNA269750, NP_002919.1, 209324_s_at
Figure 645: PRO58159
Figure 646: DNA269750, RGS16, 209325_s_at
Figure 647: PR058159
Figure 648: DNA269630, NP_003281.1, 209344_at
Figure 649: PR058042
Figure 650: DNA330170, AF109161, 209357_at
Figure 651: PRO84807
Figure 652: DNA253811, NP_004410.2, 209457_at
Figure 653: PR049214
Figure 654: DNA324899, NP_002938.1, 209507_at
Figure 655: PRO81503
Figure 656: DNA328663, NP_057157.1, 209524_at
Figure 657: PR036183
Figure 658: DNA328663, CGI-142, 209526_s_at
Figure 659: PR036183
Figure 660A-B: DNA328670, BC001618, 209610_s_at

Figure 661: PRO70011
Figure 662: DNA328599, HSNFKBS, 209636_at
Figure 663: PRO84382
Figure 664: DNA330186, NP_004327.1, 209642_at
Figure 665: PRO85434
Figure 666: DNA330191, NP_036249.1, 209715_at
Figure 667: PRO85439
Figure 668: DNA329178, HSU90552, 209770_at
Figure 669: PRO84801
Figure 670: DNA329205, NP_001343.1, 209782_s_at
Figure 671: PR084821
Figure 672: DNA226436, NP_001772.1, 209795_at
Figure 673: PR036899
Figure 674A-B: DNA196499, AB002384, 209829_at
Figure 675: PR024988
Figure 676: DNA330197, NP_112190.1, 209832_at
Figure 677: PRO85445
Figure 678: DNA331334, AF117233, 209845_at
Figure 679: PRO86418
Figure 680: DNA273915, NP_036215.1, 2098648_at

Figure 681: PR061867
Figure 682: DNA330198, AB014719, 209871_s_at
Figure 683: PRO85446
Figure 684: DNA154921, DNA154921, 209967_s_at
Figure 685A-B: DNA227134, NP_000918.1, 209993_at
Figure 686: PRO37597
Figure 687: DNA226658, NP_003736.1, 209999_x_at
Figure 688: PR037121
Figure 689: DNA226658, SSI-1, 210001_s_at
Figure 690: PRO37121
Figure 691A-B: DNA328685, NP_127497.1, 210113_s_at
Figure 692: PRO34751
Figure 693: DNA330203, NP_003755.1, 210190_at
Figure 694: PR085449
Figure 695: DNA331335, AF070576, 210202_s_at
Figure 696: DNA217253, NP_000749.1, 210229_s_at
Figure 697: PR034295
Figure 698: DNA328690, NP_524145.1, 210240_s_at
Figure 699: PR059660
Figure 700: DNA330206, NP_005801.2, 210288_at

Figure 701: PR085450
Figure 702: DNA225528, NP_000610.1, 210354_at
Figure 703: PRO35991
Figure 704: DNA331336, TANK, 210458_s_at
Figure 705: PRO86419
Figure 706: DNA329217, BC003406, 210571_s_at
Figure 707: PR084828
Figure 708: DNA330210, HSU03858, 210607_at
Figure 709: PRO126
Figure 710: DNA331337, TNFSF11, 210643_at
Figure 711: PR0206
Figure 712: DNA227446, NP_003551.1, 210647_x_at
Figure 713: PR037909
Figure 714: DNA256521, NP_038459.1, 210690_at
Figure 715: PRO51556
Figure 716: DNA331338, AF188298, 210757_x_at
Figure 717: PR086420
Figure 718: DNA237817, NP_001307.1, 210766_s_at
Figure 719: PR038923
Figure 720A-B: DNA330216, NP_006445.1, 210778_s_at

Figure 721: PR085457
Figure 722: DNA188234, NP_000630.1, 210865_at
Figure 723: PR021942
Figure 724: DNA228132, LCE, 210868_s_at
Figure 725: PR038595
Figure 726: DNA238565, MCM7, 210983_s_at
Figure 727: PR039210
Figure 728: DNA326239, NP_006752.1, 210996_s_at
Figure 729: PR039530
Figure 730: DNA325039, NP_004902.1, 211048_s_at
Figure 731: PR02733
Figure 732: DNA288254, NP_006000.2, 211058_x_at
Figure 733: PR069536
Figure 734: DNA288254, TUBA3, 211072_x_at
Figure 735: PR069536
Figure 736: DNA188234, TNFSF6, 211333_s_at
Figure 737: PR021942
Figure 738: DNA331339, B3GALT3, 211379_s_at
Figure 739: PRO1074
Figure 740A-B: DNA275066, NP_000170.1, 211450-s_at

Figure 741: PRPO62786
Figure 742: DNA331340, HUMCD26A, 211478_s_at
Figure 743: PRO80881
Figure 744: DNA226578, CCNG2, 211559_s_at
Figure 745: PRO37041
Figure 746: DNA331341, AB055881, 211583_x_at
Figure 747: PRO86421
Figure 748: DNA327760, NP_114430.1, 211685_s_at
Figure 749: PR083729
Figure 750: DNA328706, BC021909, 211714_x_at
Figure 751: PR010347
Figure 752: DNA329225, EVI2B, 211742_s_at
Figure 753: PRO84833
Figure 754: DNA328649, TUBA6, 211750_x_at
Figure 755: PRO84424
Figure 756: DNA254725, NP_002257.1, 211762_s_at
Figure 757: PR049824
Figure 758: DNA330226, AF198052, 211794_at
Figure 759: PR085463
Figure 760: DNA227173, FYB, 211795_s_at

Figure 761: PR037636
Figure 762A-B: DNA331342, DEFCAP, 211822_s_at
Figure 763: PR086422
Figure 764: DNA331343, AK026398, 211824_x_at
Figure 765: PR086423
Figure 766: DNA226881, FLI1, 211825_s_at
Figure 767: PR037344
Figure 768: DNA226176, CXCR4, 211919_s_at
Figure 769: PR036639
Figure 770: DNA272286, CAT, 211922_s_at
Figure 771: PR060544
Figure 772A-B: DNA325227, NP_005338.1, 211936_at
Figure 773: PRO81785
Figure 774A-B: DNA329227, HSRANBP5, 211953_s_at
Figure 775: PR082307
Figure 776A-C: DNA331344, 1390535.1, 211986_at
Figure 777: PR086424
Figure 778: DNA287433, NP_006810.1, 212009_s_at
Figure 779: PRO69690
Figure 780: DNA330236, 228447.20, 212071_s_at

Figure 781: PR085472
Figure 782A-B: DNA150956, BAA06685.1, 212110_at
Figure 783: PRO12560
Figure 784: DNA330240, CAA52801.1, 212141_at
Figure 785: PR085475
Figure 786: DNA330240, HSP1CDC21, 212142_at
Figure 787A-B: DNA150829, AB014568, 212144_at
Figure 788: DNA324232, NP_005991.1, 212242_at
Figure 789: PRO26228
Figure 790: DNA329182, BC016852, 212259_s_at
Figure 791: PR084805
Figure 792: DNA150980, HUMMAC30X, 212279_at
Figure 793: DNA150980, DNA150980, 212281_s_at
Figure 794: PRO12566
Figure 795: DNA253017, DNA253017, 212282_at
Figure 796: PRO48926
Figure 797: DNA328719, BC012895, 212295_s_at
Figure 798: PR084475
Figure 799: DNA271103, NP_005796.1, 212296_at
Figure 800: PR059425

Figure 801A-B: DNA331345, NP_003596.1, 212307_s_at
Figure 802: PR059330
Figure 803: DNA326808, BC019307, 212312_at
Figure 804: PRO83141
Figure 805: DNA331346, BC011685, 212330_at
Figure 806: PR062868
Figure 807: DNA331347, 170995.1, 212334_at
Figure 808: PR086425
Figure 809A-B: DNA330216, MAD4, 212346_s_at
Figure 810: PR085457
Figure 811: DNA331348, AF040963, 212347_x_at
Figure 812: PR085457
Figure 813A-B: DNA330250, NP_060727.1, 212406_s_at
Figure 814: PR085483
Figure 815: DNA330251, NP_059965.1, 212430_at
Figure 816: PR085484
Figure 817: DNA269630, TPM4, 212481_s_at
Figure 818: PR058042
Figure 819A-B: DNA328731, 234169.5, 212500_at
Figure 820: PR084484

Figure 821: DNA328732, NP_116193.1, 212502_at
Figure 822: PR084485
Figure 823: DNA226041, NP_005555.1, 212531_at
Figure 824: PR036504
Figure 825: DNA269882, HSWEE1HU, 212533_at
Figure 826: PR058280
Figure 827A-D: DNA328737, 148650.1, 212560_at
Figure 828: PR084490
Figure 829: DNA275100, DNA275100, 212589_at
Figure 830: DNA331349, BC013106, 212590_at
Figure 831: PR086426
Figure 832: DNA327776, 1379302.1, 212593_s_at
Figure 833: PR083742
Figure 834: DNA151487, DNA151487, 212594_at
Figure 835: PRO11833
Figure 836: DNA287198, K-ALPHA-1, 212639_x_at
Figure 837: PR069484
Figure 838: DNA328744, AF318364, 212680_x_at
Figure 839: PR084496
Figure 840A-B: DNA331350, NP_060903.2, 212689_s_at

Figure 841: PR086427
Figure 842: DNA273193, DNA273193, 212811_x_at
Figure 843: DNA331351, BC004298, 212823_s_at
Figure 844: DNA254940, BAA91770.1, 213008_at
Figure 845: PR050030
Figure 846A-B: DNA330275, BAA25487.1, 213045_at
Figure 847: PR085504
Figure 848: DNA323879, NP_003991.1, 213060_s_at
Figure 849: PRO80622
Figure 850A-B: DNA331352, BAA76818.1, 213088_s_at
Figure 851: PR086429
Figure 852A-B: DNA331353, AB023191, 213092_x_at Figure 853A-C: DNA329244, 979567.11, 213106_at
Figure 854: PR084849
Figure 855: DNA328567, BTG3, 213134_x_at
Figure 856: PRO84364
Figure 857: DNA330277, CAB45152.1, 213142_x_at
Figure 858: PR085506
Figure 859A-B: DNA329248, BAA20816.1, 213302_at
Figure 860: PRO84850

Figure 861A-B: DNA331354, PPP2R5C, 213305_s_at
Figure 862: PR086430
Figure 863: DNA155418, DNA155418, 213326_at
Figure 864A-B: DNA331355, AAG24545.1, 213330_s_at
Figure 865: PR086431
Figure 866: DNA331356, CASP8, 213373_s_at
Figure 867: PRO86432
Figure 868: DNA106195, DNA106195, 213454_at
Figure 869: DNA327795, BC014226, 213457_at
Figure 870: DNA328766, NP_006077.1, 213476_x_at
Figure 871: PRO84514
Figure 872: DNA227483, SQLE, 213562_s_at
Figure 873: PR037946
Figure 874: DNA327800, 1251176.10, 213575_at
Figure 875: PR083763
Figure 876: DNA159560, DNA159560, 213577_at
Figure 877: DNA331357, BC010494, 213646_x_at
Figure 878: PR038556
Figure 879: DNA330293, BC011922, 213666_at
Figure 880: PR085520

Figure 881: DNA325704, MARS, 213671_s_at
Figure 882: PR082188
Figure 883: DNA328629, TUBB2, 213726_x_at
Figure 884: PR084407
Figure 885: DNA328771, HSMYOSIE, 213733_at
Figure 886: DNA150713, EBP, 2137877_s_at
Figure 887: PRO12082
Figure 888: DNA330295, NP_037515.1, 213951_s_at
Figure 889: PRO85522
Figure 890: DNA331358, NP_037445.1, 213996_at
Figure 891: PR086433
Figure 892: DNA329136, HSPC111, 214011_s_at
Figure 893: PR084772
Figure 894: DNA270687, DNA270687, 214032_at
Figure 895: DNA227224, LC27, 214039_s_at
Figure 896: PR037687
Figure 897: DNA330298, BC011911, 214095_at
Figure 898: PRO83772
Figure 899: DNA329254, BC004215, 214096_s_at
Figure 900: PR084854

Figure 901A-B: DNA331359, 332730.12, 214155_s_at
Figure 902: PR086434
Figure 903: DNA331360, AK022497, 214177_s_at
Figure 904: PRO86435

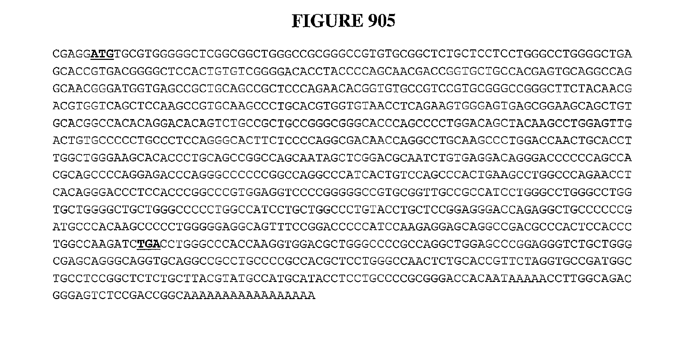

Figure 905: DNA331361, NP_003318.1, 214228_x_at

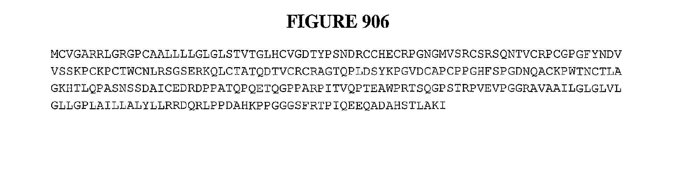

Figure 906: PR02398
Figure 907: DNA331362, AF275719, 214359_s_at
Figure 908: PRO86436
Figure 909: DNA326089, NP_000508.1, 214414_x_at
Figure 910: PRO3629
Figure 911: DNA271374, CHAF1A, 214426_x_at
Figure 912: PR059673
Figure 913: DNA327811, SHMT2, 214437_s_at
Figure 914: PRO83772
Figure 915: DNA331363, AF001383, 214439_x_at
Figure 916: PRO86437
Figure 917: DNA150971, NP_002249.1, 214470_at
Figure 918: PRO12564
Figure 919: DNA331364, CREM, 214508_x_at
Figure 920: PRO86438

Figure 921: DNA216515, NP_003166.1, 214567_s_at
Figure 922: PR034267
Figure 923: DNA330303, BAA05499.1, 214662_at
Figure 924: PRO85528
Figure 925: DNA330308, 307914.1, 215029_at
Figure 926: PRO85533
Figure 927: DNA196372, HSBCLXL, 215037_s_at
Figure 928: PR024874
Figure 929: DNA270522, NP-006013.1, 215111_s_at
Figure 930: PR058899
Figure 931: DNA330314, 026641.5, 215275_at
Figure 932: PRO85538
Figure 933: DNA328801, 407831.1, 215392_at
Figure 934: PRO84543
Figure 935A-B: DNA331134, NP_003381.1, 215711_s_at
Figure 936: PRO86275
Figure 937A-B: DNA256461, NP_009017.1, 216228_s_at
Figure 938: PR051498
Figure 939: DNA329266, BC000142, 216237_s_at
Figure 940: PRO12845

Figure 941: DNA88296, NP_005733.1, 216640_s_at
Figure 942: PRO2274
Figure 943: DNA225720, AF151900, 216693_x_at
Figure 944: PR036183
Figure 945A-B: DNA66475, NP_004439.1, 216836_s_at
Figure 946: PRO1204
Figure 947: DNA331365, NP_004168.1, 216985_s_at
Figure 948: PRO86439
Figure 949: DNA331366, HUMGPCR, 217028_at
Figure 950: PR04516
Figure 951A-B: DNA331367, BAA34514.1, 217100_s_at
Figure 952: PR086440
Figure 953: DNA331368, NP_112233.1, 217226_s_at
Figure 954: PR086441
Figure 955: DNA331369, HSU88968, 217294_s_at
Figure 956: DNA328819, NP_057145.1, 217783_s_at
Figure 957: PR084557
Figure 958: DNA328303, NP_056525.1, 217807_s_at
Figure 959: PR084173
Figure 960: DNA227172, NP_066952.1, 217848_s_at

Figure 961: PR037635
Figure 962: DNA330345, NP_055130.1, 217906_at
Figure 963: PR085566
Figure 964: DNA227218, NP_003721.2, 217983_s_at
Figure 965: PR037681
Figure 966: DNA227218, RNASE6PL, 217984_at
Figure 967: PR037681
Figure 968: DNA328831, NP-057329.1, 217989_at
Figure 969: PR0233
Figure 970: DNA328833, BC018929, 217996_at
Figure 971: PR084569
Figure 972: DNA328834, AF220656, 217997_at
Figure 973: DNA273008, NP_003972.1, 218009_s_at
Figure 974: PRO61079
Figure 975: DNA328837, NP_057149.1, 218046_s_at
Figure 976: PR081876
Figure 977: DNA329276, NP_077001.1, 218069_at
Figure 978: PRO12104
Figure 979: DNA330356, NP_006318.1, 218118_s_at
Figure 980: PR085572

Figure 981: DNA327857, NP_057386.1, 218142_s_at
Figure 982: PRO83799
Figure 983: DNA327858, NP_036473.1, 218238_at
Figure 984: PRO83800
Figure 985: DNA328850, NP_057187.1, 218254_s_at
Figure 986: PR084581
Figure 987A-B: DNA331370, NP_055101.2, 218266_s_at
Figure 988: PRO11501
Figure 989: DNA331371, NP_060401.1, 218272_at
Figure 990: PRO86442
Figure 991: DNA331372, FLJ20950, 218298_s_at
Figure 992: PR086443
Figure 993: DNA327862, NP_060445.1, 218349_s_at
Figure 994: PR083803
Figure 995: DNA328854, NP_056979.1, 218350_s_at
Figure 996: PR084585
Figure 997: DNA304468, NP_077300.1, 218358_at
Figure 998: PRO71044
Figure 999: DNA328856, NP_068376.1, 218380_at
Figure 1000: PRO84586

Figure 1001: DNA327865, NP_079105.1, 218454_at
Figure 1002: PR083806
Figure 1003: DNA329286, NP_005691.2, 218567_x_at
Figure 1004: PR069644
Figure 1005A-B: DNA273435, NP_057532.1, 218585_s_at
Figure 1006: PR061430
Figure 1007: DNA330377, NP_036577.1, 218638_s_at
Figure 1008: PRO85590
Figure 1009: DNA304835, NP_071327.1, 218681_s_at
Figure 1010: PR071242
Figure 1011: DNA329288, NP_061910.1, 218695_at
Figure 1012: PRO84880
Figure 1013: DNA326185, FLJ13912, 218719_s_at
Figure 1014: PRO82602
Figure 1015: DNA330381, NP_076958.1, 218741_at
Figure 1016: PR038668
Figure 1017: DNA331373, NP_075526.1, 218769_s_at
Figure 1018: PRO86444
Figure 1019A-B: DNA327971, NP_006104.3, 218807_at
Figure 1020: PR083893

Figure 1021: DNA330388, NP_078905.1, 218883_s_at
Figure 1022: PRO85597
Figure 1023: DNA331092, NP_078918.2, 218885_s_at
Figure 1024: PRO86243
Figure 1025: DNA226633, NP_060376.1, 218886_at
Figure 1026: PR037096
Figure 1027: DNA328881, NP_057706.2, 218890_x_at
Figure 1028: PR049469
Figure 1029: DNA325622, NP_060518.1, 218894_s_at
Figure 1030: PRO82113
Figure 1031: DNA328364, SIGIRR, 218921_at
Figure 1032: PR084223
Figure 1033: DNA329050, NP_057053.1, 218982_s_at
Figure 1034: PR084712
Figure 1035: DNA330391, NP_076999.1, 219000_s_at
Figure 1036: PR034008
Figure 1037: DNA227187, NP_057703.1, 219014_at
Figure 1038: PR037650
Figure 1039: DNA329293, NP_057136.1, 219037_at
Figure 1040: PR084883

Figure 1041: DNA331374, NP_009177.2, 219157_at
Figure 1042: PR086445
Figure 1043: DNA331375, AK000192, 219168_s_at
Figure 1044: PR086446
Figure 1045: DNA329223, NP_037517.1, 219183_s_at
Figure 1046: PR084831
Figure 1047: DNA329296, NP_060328.1, 219258_at
Figure 1048: PR084886
Figure 1049A-B: DNA331376, NP_079484.1, 219304_s_at
Figure 1050: PRO86447
Figure 1051: DNA287404, NP_073748.1, 219334_s_at
Figure 1052: PR069661
Figure 1053: DNA331377, NP_060753.1, 219347_at
Figure 1054: PR086448
Figure 1055: DNA254518, NP_057354.1, 219371_s_at
Figure 1056: PR049625
Figure 1057: DNA328902, NP_071750.1, 219452_at
Figure 1058: PR084623
Figure 1059: DNA327890, NP_079021.1, 219493_at
Figure 1060: PRO83826

Figure 1061A-B: DNA227179, NP_059120.1, 219505_at
Figure 1062: PR037642
Figure 1063: DNA329299, NP_004660.1, 219529_at
Figure 1064: PRO84888
Figure 1065: DNA330410, NP_060925.1, 219555_s_at
Figure 1066: PR085618
Figure 1067: DNA327891, NP_078909.1, 219563_at
Figure 1068: PR083827
Figure 1069: DNA331378, ALS2CR8, 219834_at
Figure 1070: PR086449
Figure 1071: DNA255255, LOC64116, 219869_s_at
Figure 1072: PR050332
Figure 1073: DNA256325, NP_005470.1, 219889_at
Figure 1074: PRO51367
Figure 1075: DNA328923, NP_075379.1, 219892_at
Figure 1076: PR084640
Figure 1077: DNA330421, NP_057438.2, 219911_s_at
Figure 1078: PRO85626
Figure 1079: DNA330423, NP_037466.2, 219920_s_at
Figure 1080: PR085628

Figure 1081: DNA328924, NP_057150.2, 219933_at
Figure 1082: PR084641
Figure 1083: DNA330537, NP_060533.2, 220085_at
Figure 1084: PR081892
Figure 1085: DNA227302, NP_037401.1, 220132_s_at
Figure 1086: PR037765
Figure 1087A-B: DNA328930, NP_038465.1, 220253_s_at
Figure 1088: PR023525
Figure 1089: DNA330436, NP_037394.1, 220319_s_at
Figure 1090: PR085639
Figure 1091: DNA327904, NP_071419.2, 220330_s_at
Figure 1092: PRO83839
Figure 1093: DNA331379, PHEMX, 220558_x_at
Figure 1094: PR086450
Figure 1095: DNA330440, NP_079098.1, 220591_s_at
Figure 1096: PRO85642
Figure 1097: DNA255734, NP_057607.1, 220646_s_at
Figure 1098: PRO50791
Figure 1099A-B: DNA327908, MCM10, 220651_s_at
Figure 1100: PRO83843

Figure 1101: DNA323756, NP_057267.2, 220688_s_at
Figure 1102: PRO80512
Figure 1103: DNA331380, DKFZp566O084Homo, 220690_s_at
Figure 1104: DNA288247, NP_478059.1, 220892_s_at
Figure 1105: PR070011
Figure 1106: DNA331381, BA108L7.2, 220974_x_at
Figure 1107: PRO86451
Figure 1108: DNA287397, NP_112214.1, 220987_s_at
Figure 1109: PR069654
Figure 1110: DNA328945, NP_079177.2, 221081_s_at
Figure 1111: PR084657
Figure 1112: DNA331382, CISH, 221223_x_at
Figure 1113: PR086452
Figure 1114: DNA330451, NP_110429.1, 221249_s_at
Figure 1115: PR085652
Figure 1116: DNA328948, NP_110437.1, 221253_s_at
Figure 1117: PR084659
Figure 1118: DNA326507, NP-1 12490.2,221267-s-at
Figure 1119: PRO82879
Figure 1120: DNA329471, NP_110387.1, 221417_x_at
Figure 1121: PR085036
Figure 1122A-B: DNA329314, 1149046.7, 221478_at
Figure 1123: PRO84902
Figure 1124: DNA227303, NP_004322.1, 221479_s_at
Figure 1125: PR037766
Figure 1126: DNA326221, NP_057179.1, 221521_s_at
Figure 1127: PRO82634
Figure 1128: DNA330457, NP_076944.1, 221559_s_at
Figure 1129: PR085658
Figure 1130: DNA329318, IRO033793, 221564_at

Figure 1131: DNA329319, BC006401, 221601_s_at
Figure 1132: PRO1607
Figure 1133: DNA329319, NP_005440.1, 221602_s_at
Figure 1134: PRO1607
Figure 1135: DNA330459, NP_060083.1, 221677_s_at
Figure 1136: PR050083
Figure 1137: DNA328961, NP_443112.1, 221756_at
Figure 1138: PR084667
Figure 1139: DNA328961, MGC17330, 221757_at
Figure 1140: PR084667
Figure 1141: DNA331383, BC007588, 221769_at
Figure 1142: DNA331384, AK026326, 221985_at
Figure 1143: PRO86454
Figure 1144: DNA330467, NP_060114.1, 221986_s_at
Figure 1145: PR085665
Figure 1146: DNA254739, NP_068766.1, 221987_s_at
Figure 1147: PR049837
Figure 1148: DNA257797, DNA257797, 222036_s_at
Figure 1149: DNA257798, DNA257798, 222037_at
Figure 1150: DNA325648, NP_037409.2, 222077_s_at
Figure 1151: PRO82139
Figure 1152A-B: DNA331385, AF274889S4, 222088_s_at
Figure 1153: DNA331386, HST000012, 222150_s_at
Figure 1154A-B: DNA331387, NP_008919.2, 222162_s_at
Figure 1155: PRO86456
Figure 1156: DNA328977, NP_071344.1, 222216_s_at
Figure 1157: PRO84678
Figure 1158: DNA325821, BC014334, 222402_at

Figure 1159: PR082287
Figure 1160A-B: DNA256489, NP_079110.1, 222464_s_at
Figure 1161: PRO51526
Figure 1162: DNA304460, BC003048, 222500_at
Figure 1163: PR04984
Figure 1164: DNA327942, NP_060596.1, 222642_s_at
Figure 1165: PRO83870
Figure 1166: DNA327943, NP_055399.1, 222646_s_at
Figure 1167: PRO865
Figure 1168A-B: DNA273435, RAMP, 222680_s_at
Figure 1169: PR061430
Figure 1170: DNA330486, HSM802473, 222692_s_at
Figure 1171: DNA331388, NP_068747.1, 222753_s_at
Figure 1172: PRO3567
Figure 1173: DNA329335, AK023411, 222843_at
Figure 1174: PRO84919
Figure 1175: DNA331389, NP_071428.2, 222848_at
Figure 1176: PRO81238
Figure 1177: DNA287404, FLJ22833, 222872_x_at

Figure 1178: PR069661
Figure 1179: DNA330500, AK022872, 222889_at
Figure 1180: PR085693
Figure 1181A-B: DNA287236, AB024334, 222985_at
Figure 1182: PRO10607
Figure 1183: DNA329571, NP_057547.3, 222996_s_at
Figure 1184: PR051662
Figure 1185: DNA189412, NP_057390.1, 223054_at
Figure 1186: PR025349
Figure 1187: DNA288247, PSA, 223062_s_at
Figure 1188: PR070011
Figure 1189: DNA331390, AAF28975.1, 223070_at
Figure 1190: PRO86457
Figure 1191: DNA324209, NP_057018.1, 223096_at
Figure 1192: PR080902
Figure 1193: DNA330436, MIR, 223129_x_at
Figure 1194: PR085639
Figure 1195: DNA330513, AF212221, 223130_s_at
Figure 1196: PRO85703
Figure 1197: DNA254276, NP_054896.1, 223180_s_at
Figure 1198: PR049387
Figure 1199: DNA254416, NP_060915.1, 223209_s_at
Figure 1200: PRO49526
Figure 1201A-B: DNA257461, NP_113607.1, 223217_s_at
Figure 1202: PR052040
Figure 1203A-B: DNA257461, MAIL, 223218_s_at

Figure 1204: PR052040
Figure 1205: DNA326056, NP_072088.1, 223264_at
Figure 1206: PR082491
Figure 1207: DNA330518, BC002493, 223274_at
Figure 1208: PR085708
Figure 1209: DNA329355, NP_150596.1, 223299_at
Figure 1210: PR050434
Figure 1211: DNA227125, AF132297, 223377_x_at
Figure 1212: PR037588
Figure 1213A-B: DNA331391, NP_065947.1, 223464_at
Figure 1214: PR049998
Figure 1215: DNA329456, NP_057126.1, 223490_s_at
Figure 1216: PR085023
Figure 1217: DNA330536, NP_115666.1, 223542_at
Figure 1218: PR085722
Figure 1219: DNA330537, AF155827, 223556_at
Figure 1220: PR081892
Figure 1221: DNA287253, LOC85028, 223773_s_at
Figure 1222: PR069527
Figure 1223: DNA327200, NP_114156.1, 223836_at
Figure 1224: PRO1065
Figure 1225: DNA331392, NP_004186.1, 223851_s_at

Figure 1226: PR0364
Figure 1227: DNA331393, D83532, 223961_s_at
Figure 1228: PR086458
Figure 1229: DNA330552, BC001104, 223984_s_at
Figure 1230: PR085736
Figure 1231: DNA330558, NP_057588.1, 224330_s_at
Figure 1232: PR084950
Figure 1233: DNA328323, NP_114148.2, 224428_s_at
Figure 1234: PR069531
Figure 1235: DNA331394, MGC11316, 224482_s_at
Figure 1236: PRO86459
Figure 1237: DNA327976, NP_116120.1, 224511_s_at
Figure 1238: PR069574
Figure 1239: DNA329374, NP_115735.1, 224523_s_at
Figure 1240: PR084953
Figure 1241: DNA331395, TNFRSF18, 224553_s_at
Figure 1242: PR086460
Figure 1243: DNA331396, 1357555.1, 224603_at
Figure 1244: PR086461
Figure 1245A-B: DNA327981, 344095.3, 224654_at
Figure 1246: PRO83901
Figure 1247A-B: DNA330574, AB033054, 224698_at

Figure 1248: DNA324173, NP_115766.2, 224714_at
Figure 1249: PR080871
Figure 1250: DNA257352, DNA257352, 224739_at
Figure 1251: PR051940
Figure 1252A-B: DNA287330, BAA86479.1, 224799_at
Figure 1253: PR069594
Figure 1254A-B: DNA287330, AB032991, 224801_at
Figure 1255: DNA331397, AK001723, 224802_at
Figure 1256: PR023259
Figure 1257A-C: DNA329379, 010205.2, 224847_at
Figure 1258: PR084957
Figure 1259: DNA257789, NP_116219.1, 224903_at
Figure 1260: PR052338
Figure 1261: DNA151170, DNA151170, 224989_at
Figure 1262: PRO12626
Figure 1263A-B: DNA327993, 898436.7, 225133_at
Figure 1264: PR081138
Figure 1265: DNA327996, BC010181, 225195_at
Figure 1266: PR083915
Figure 1267: DNA329394, BC010416, 225201_s_at
Figure 1268: DNA329396, NP_060866.1, 225253_s_at
Figure 1269: PR084972
Figure 1270: DNA304802, AAH00967.1, 225439_at
Figure 1271: PR071212
Figure 1272A-B: DNA330617, 336147.2, 225447_at

Figure 1273: PR059923
Figure 1274: DNA196561, DNA196561, 225470_at
Figure 1275A-B: DNA331398, 234824.7, 225478_at
Figure 1276: PR086462
Figure 1277: DNA329405, HSM800962, 225520_at
Figure 1278A-B: DNA330620, CAB55950.1, 225533_at
Figure 1279: PRO85794
Figure 1280: DNA330621, AF116628, 225535_s_at
Figure 1281: DNA328008, 240051.4, 225541_at
Figure 1282: PR083926
Figure 1283: DNA329406, 1503139.10, 225562_at
Figure 1284: PRO84979
Figure 1285: DNA304469, NP_149078.1, 225621_at
Figure 1286: PR071045
Figure 1287: DNA331399, 994419.37, 225622_at
Figure 1288: PR086463
Figure 1289A-B: DNA331400, NP_060910.2, 225626_at
Figure 1290: PR086464
Figure 1291: DNA329940, NP_001805.1, 225647_s_at
Figure 1292: PR02679
Figure 1293A-B: DNA288261, NP_037414.2, 225655_at
Figure 1294: PRO70021
Figure 1295A-B: DNA331401, 336865.4, 225700_at
Figure 1296: PRO86465
Figure 1297: DNA304821, BC011254, 225706_at
Figure 1298: DNA254820, DNA254820, 225707_at
Figure 1299: PR049916

Figure 1300: DNA330633, BC003515, 225723_at
Figure 1301: DNA329417, 411336.1, 225842_at
Figure 1302: PR084989
Figure 1303A-B: DNA331402, 197159.1, 225845_at
Figure 1304: PRO86466
Figure 1305: DNA287370, BAB14983.1, 225866_at
Figure 1306: PRO69630
Figure 1307A-B: DNA331403, TP53INP1, 225912_at
Figure 1308: PRO86467
Figure 1309A-B: DNA331405, 979005.2, 225956_at
Figure 1310: PR086468
Figure 1311: DNA328021, BC004538, 226038_at
Figure 1312A-B: DNA329428, 1446144.8, 226218_at
Figure 1313: PRO84999
Figure 1314: DNA193896, DNA193896, 226276_at
Figure 1315: PR023314
Figure 1316: DNA328028, NP_005773.1, 226319_s_at
Figure 1317: PR083945
Figure 1318: DNA328028, ALY, 226320_at
Figure 1319: PR083945
Figure 1320A-B: DNA331406, 399773.27, 226334_s_at
Figure 1321: PRO86470
Figure 1322A-B: DNA331407, 198233.1, 226352_at
Figure 1323: PR086471
Figure 1324A-B: DNA331409, AB051464, 226370_at
Figure 1325A-B: DNA330675, 177663.2, 226372_at
Figure 1326: PRO85847
Figure 1327: DNA330677, 1384190.6, 226390_at
Figure 1328: PRO85849
Figure 1329: DNA331410, HSM802051, 226416_at
Figure 1330: PR086474

Figure 1331: DNA330679, BC013040, 226456-at
Figure 1332A-B: DNA330680, BC022792, 226481_at
Figure 1333: PR085852
Figure 1334: DNA330684, 984114.1, 226548_at
Figure 1335: PR085855
Figure 1336: DNA257914, DNA257914, 226743_at
Figure 1337: PRO52447
Figure 1338: DNA328038, 216863.2, 226811_at
Figure 1339: PR083953
Figure 1340: DNA328044, 039170.3, 226936_at
Figure 1341: PR083958
Figure 1342A-B: DNA330705, 198782.1, 227020_at
Figure 1343: PR085876
Figure 1344A-B: DNA330706, AF445027, 227027_at
Figure 1345: PR085877
Figure 1346: DNA331411, 232146.1, 227200_at
Figure 1347: PR086475
Figure 1348: DNA330715, BC022374, 227212_s_at
Figure 1349: PR085886
Figure 1350: DNA331412, 1378353.1, 227223_at
Figure 1351: PR086476
Figure 1352A-B: DNA329442, AH007300S2, 227265_at
Figure 1353: DNA329444, BC017821, 227278_at
Figure 1354: PRO85012
Figure 1355: DNA330718, 025465.3, 227295_at
Figure 1356: PR085889
Figure 1357A-B: DNA330721, 198680.1, 227350_at
Figure 1358: PR085892
Figure 1359: DNA226872, NP_001955.1, 227404_s_at
Figure 1360: PR037335
Figure 1361: DNA329450, BC017226, 227726_at
Figure 1362: PR085018
Figure 1363: DNA59606, DNA59606, 227803_at
Figure 1364: PRO1107
Figure 1365: DNA329456, RRP40, 227916_x_at
Figure 1366: PR085023

Figure 1367: DNA330745, BC01 1716, 228069_at
Figure 1368: PR085913
Figure 1369: DNA329460, BC017117, 228092_at
Figure 1370: PR085027
Figure 1371: DNA330436, AF187016, 228098_s_at
Figure 1372: PR085639
Figure 1373: DNA329461, BC016615, 228113_at
Figure 1374: PR085028
Figure 1375: DNA331413, 286318.11, 228284_at
Figure 1376: PR086477
Figure 1377: DNA331414, 1450017.11, 228559_at
Figure 1378: PR086478
Figure 1379: DNA331415, 345279.19, 228788_at
Figure 1380: PR086479
Figure 1381: DNA330780, 335374.1, 228955_at
Figure 1382: PR085944
Figure 1383: DNA330784, 233595.21, 228990_at
Figure 1384: PR085948
Figure 1385: DNA330787, 349981.7, 229041_s_at
Figure 1386: PR085951
Figure 1387: DNA327307, AF442769, 229215_at
Figure 1388: PR083560
Figure 1389: DNA287421, 234832.1, 229437_at
Figure 1390: PR069678
Figure 1391: DNA330799, 481875.1, 229551_x_at
Figure 1392: PRO85963
Figure 1393A-B: DNA330809, 336997.1, 229844_at
Figure 1394: PRO85973

Figure 1395: DNA329466, AF327346, 230069_at
Figure 1396: PR023814
Figure 1397A-B: DNA331416, FREQ, 230146_s_at
Figure 1398: PRO11501
Figure 1399: DNA329468, BC011589, 230170_at
Figure 1400: PRO88
Figure 1401: DNA330818, 212282.1, 230304_at
Figure 1402: PR085982
Figure 1403: DNA257756, DNA257756, 230405_at
Figure 1404: DNA329471, EDG8, 230464_at
Figure 1405: PR085036
Figure 1406: DNA331417, 333493.3, 230748_at
Figure 1407: PR086480
Figure 1408: DNA331418, 7693630.2, 230917_at
Figure 1409: PR086481
Figure 1410A-B: DNA287217, DNA287217, 231259_s_at
Figure 1411: PR036766
Figure 1412: DNA330843, 201388.1, 231832_at
Figure 1413: PR086006
Figure 1414: DNA331419, 085942.3, 232001_at
Figure 1415: PR086482
Figure 1416: DNA331420, 029520.1, 232210_at
Figure 1417: PRO86483
Figure 1418: DNA331421, 1448461.1, 232614_at
Figure 1419: PRO86484
Figure 1420: DNA328194, 998827.1, 233068_at
Figure 1421: PR084097
Figure 1422: DNA287404, AK026486, 233085_s_at
Figure 1423: PR069661

Figure 1424: DNA331422, 077853.1, 233289_at
Figure 1425: PRO86485
Figure 1426: DNA331423, AF176071, 233467_s_at
Figure 1427: DNA329571, HSPC195, 233955_x_at
Figure 1428: PR051662
Figure 1429: DNA331424, LOC112840, 235025_at
Figure 1430: PR086486
Figure 1431: DNA330888, 7687712.2, 235088_at
Figure 1432: PR069581
Figure 1433: DNA330891, AK027315, 235113_at
Figure 1434: PR086052
Figure 1435A-C: DNA331425, 228001.3, 235116_at
Figure 1436: PR020128
Figure 1437: DNA328146, BC019239, 235117_at
Figure 1438: PR084051
Figure 1439: DNA330906, NP_116171.2, 235458_at
Figure 1440: PRO86067
Figure 1441: DNA194081, DNA194081, 235556_at
Figure 1442: PR023477
Figure 1443: DNA330916, 234580.1, 235670_at
Figure 1444: PR086077
Figure 1445: DNA330943, 1042935.2, 237009_at
Figure 1446: PR086104
Figure 1447: DNA331426, 361450.1, 237542_at
Figure 1448: PRO86487
Figure 1449: DNA331427, AB052906, 238542_at
Figure 1450: PR0791
Figure 1451: DNA258952, DNA258952, 239901_at
Figure 1452: DNA328206, 1384214.3, 240277_at
Figure 1453: PR084109
Figure 1454: DNA331428, 7692702.1, 241803_s_at
Figure 1455: PR086488
Figure 1456: DNA329506, NP_387510.1, 241937_s_at
Figure 1457: PR085067
Figure 1458: DNA331429, NP_110403.1, 242020_s_at
Figure 1459: PRO86489

Figure 1460: DNA331030, 407930.2, 242648_at
Figure 1461: PR086188
Figure 1462: DNA331037, 206873.1, 242890_at
Figure 1463: PR086195
Figure 1464: DNA329507, 407430.1, 242943_at
Figure 1465: PR085068
Figure 1466: DNA331043, 005042.1, 243134_at
Figure 1467: PR086201
Figure 1468: DNA331053, 243689.1, 243509_at
Figure 1469: PR086211
Figure 1470: DNA331430, 030957.1, 243808_at
Figure 1471: PR086490
Figure 1472: DNA331431, 201839.1, 243840_at
Figure 1473: PR086491
Figure 1474: DNA331432, 151634.1, 244035_at
Figure 1475: PRO86492
Figure 1476: DNA331433, 020071.1, 244434_at
Figure 1477: PRO86493

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (e.g., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table 1 below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62. In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1 % bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass

(e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, *etc*.

The term "Natural Killer cell mediated disease" means a disease in which NK cells directly or indirectly mediate or otherwise contribute to a morbidity in a mammal. The NK cell mediated disease may be associated with cell mediated effects, lymphokine mediated effects, *etc.,* and even effects associated with other immune cells if the cells are involved.

Examples of immune-related and inflammatory diseases, some of which are immune mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease. Infectious diseases including viral diseases such as AIDS (HIV infection), hepatitis A, B, C, D, and E, herpes, *etc*., bacterial infections, fungal infections, protozoal infections and parasitic infections.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e.g*., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g*., paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in. vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -y; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12, IL-15 and tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i.e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

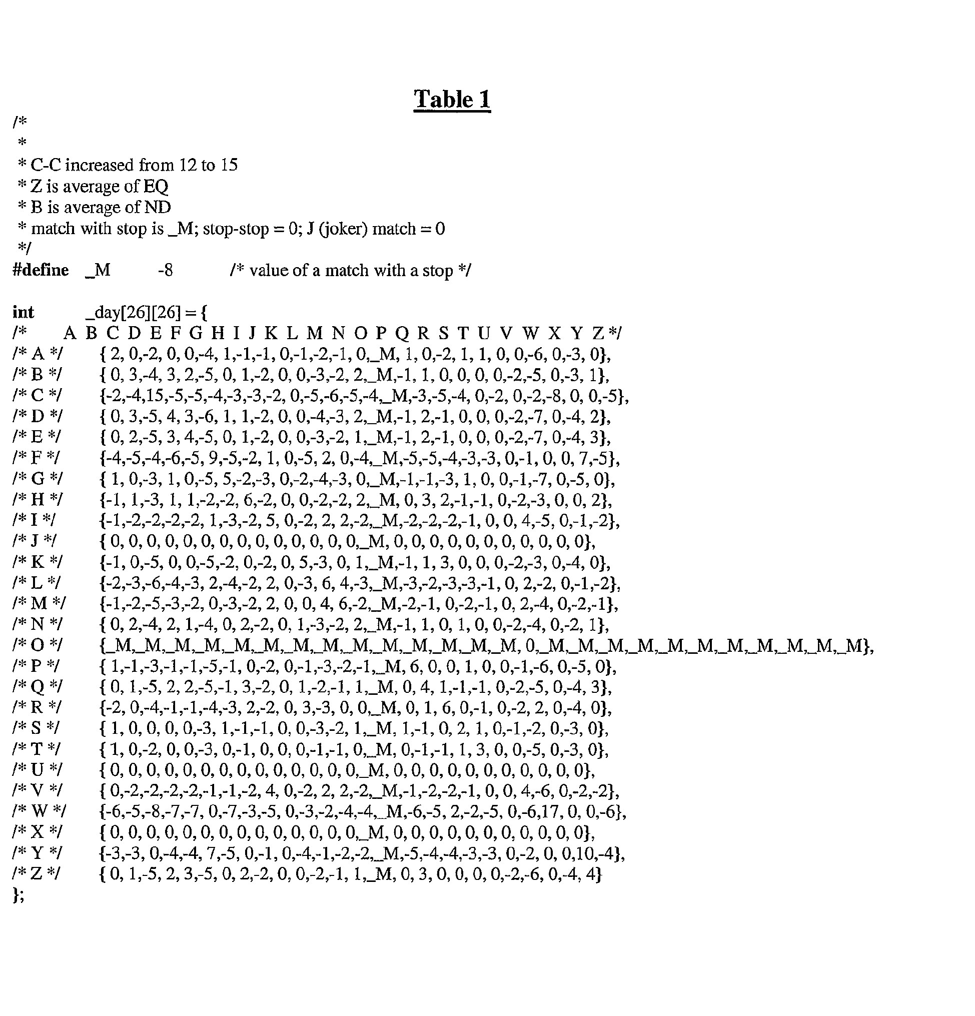

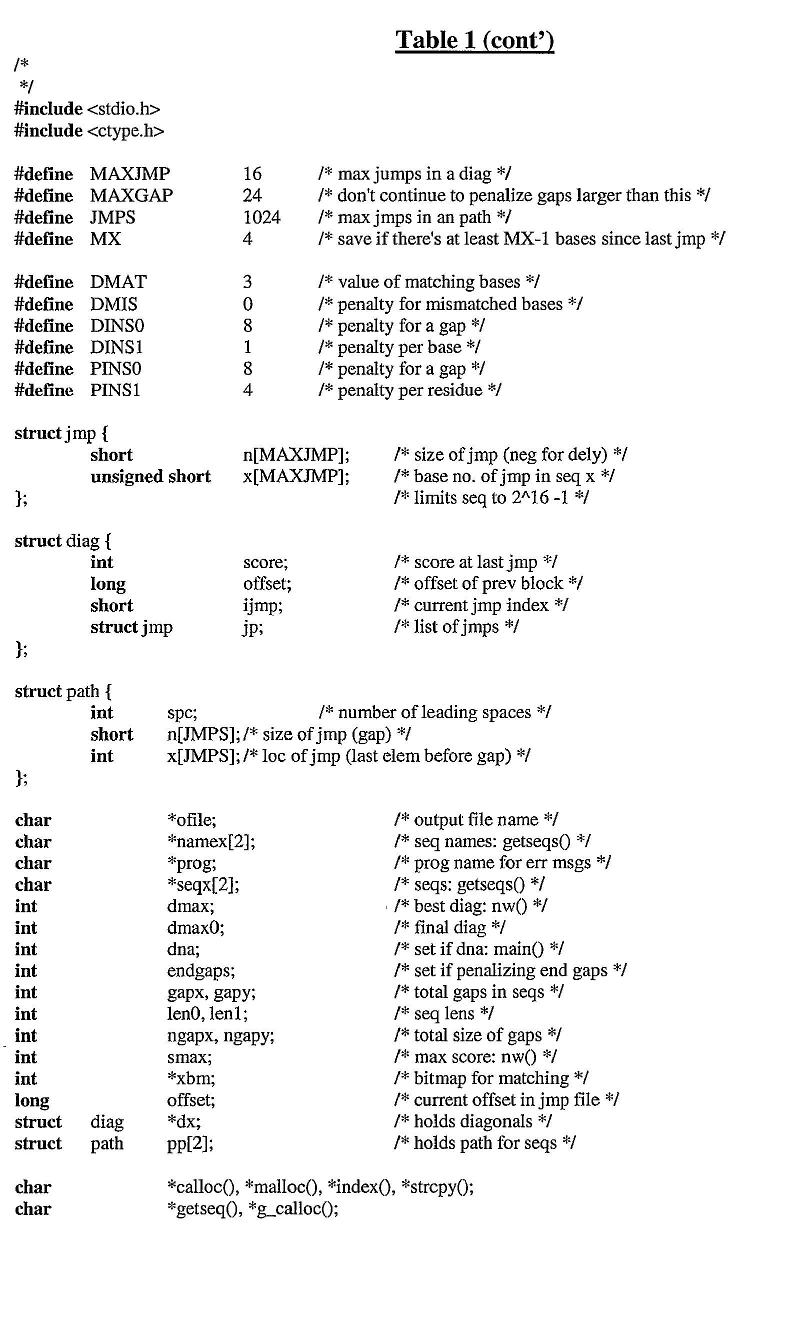

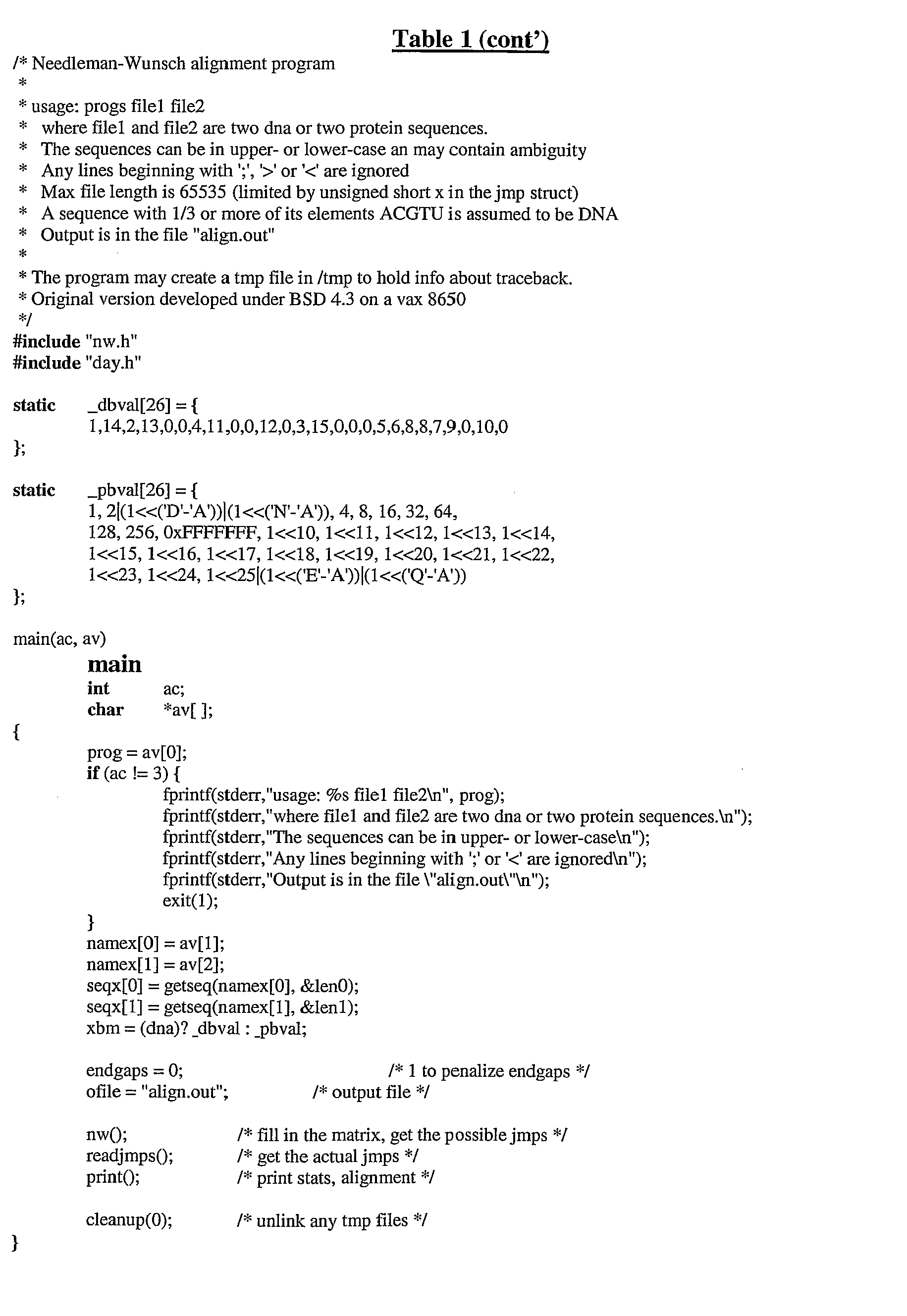

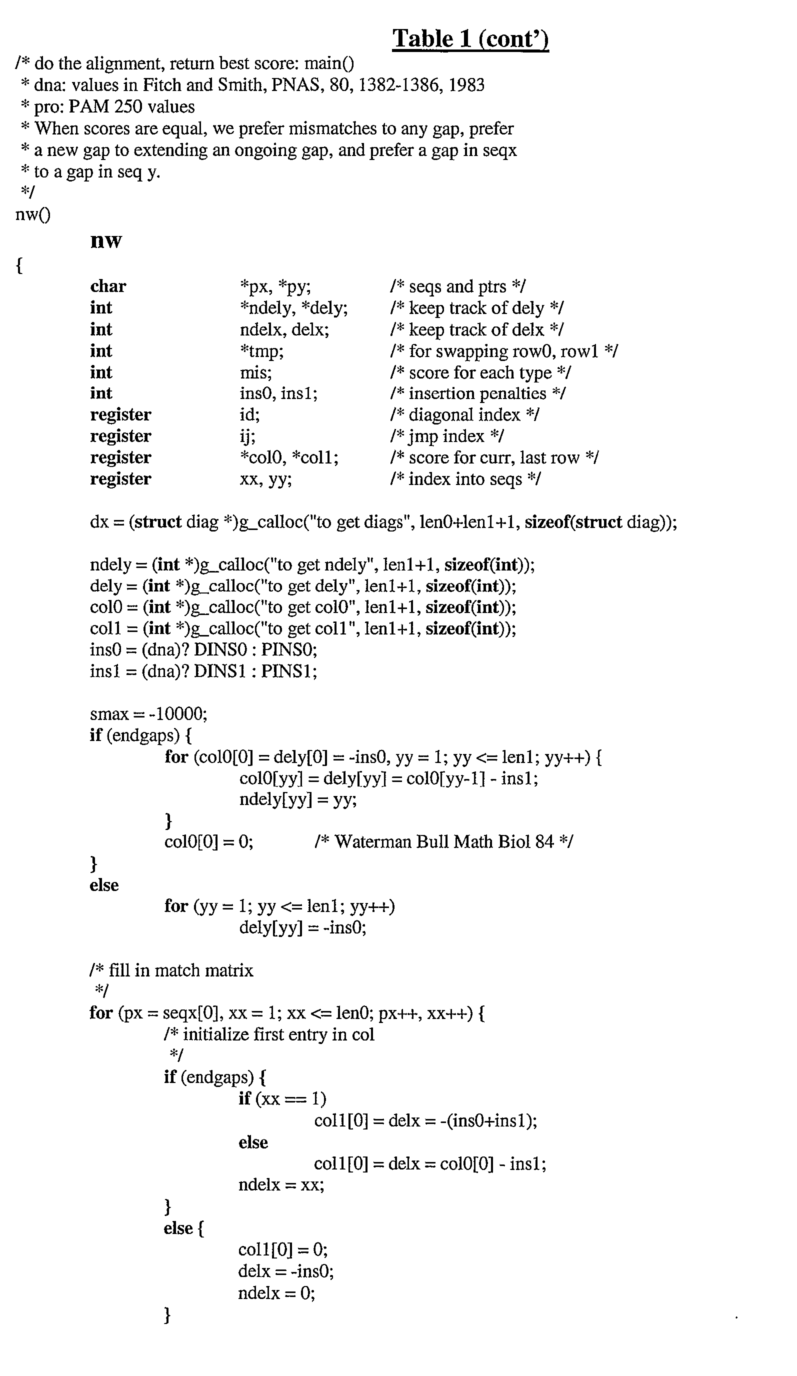

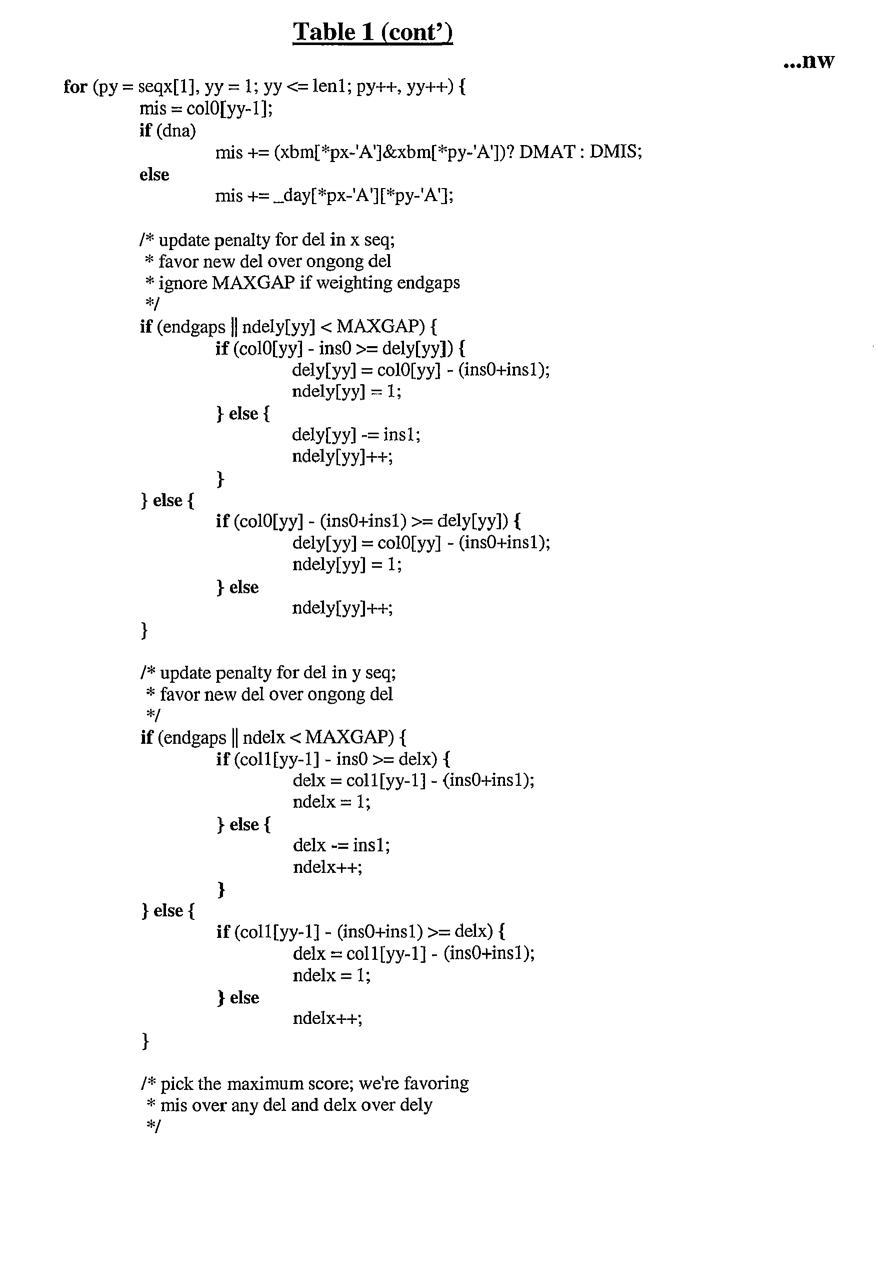

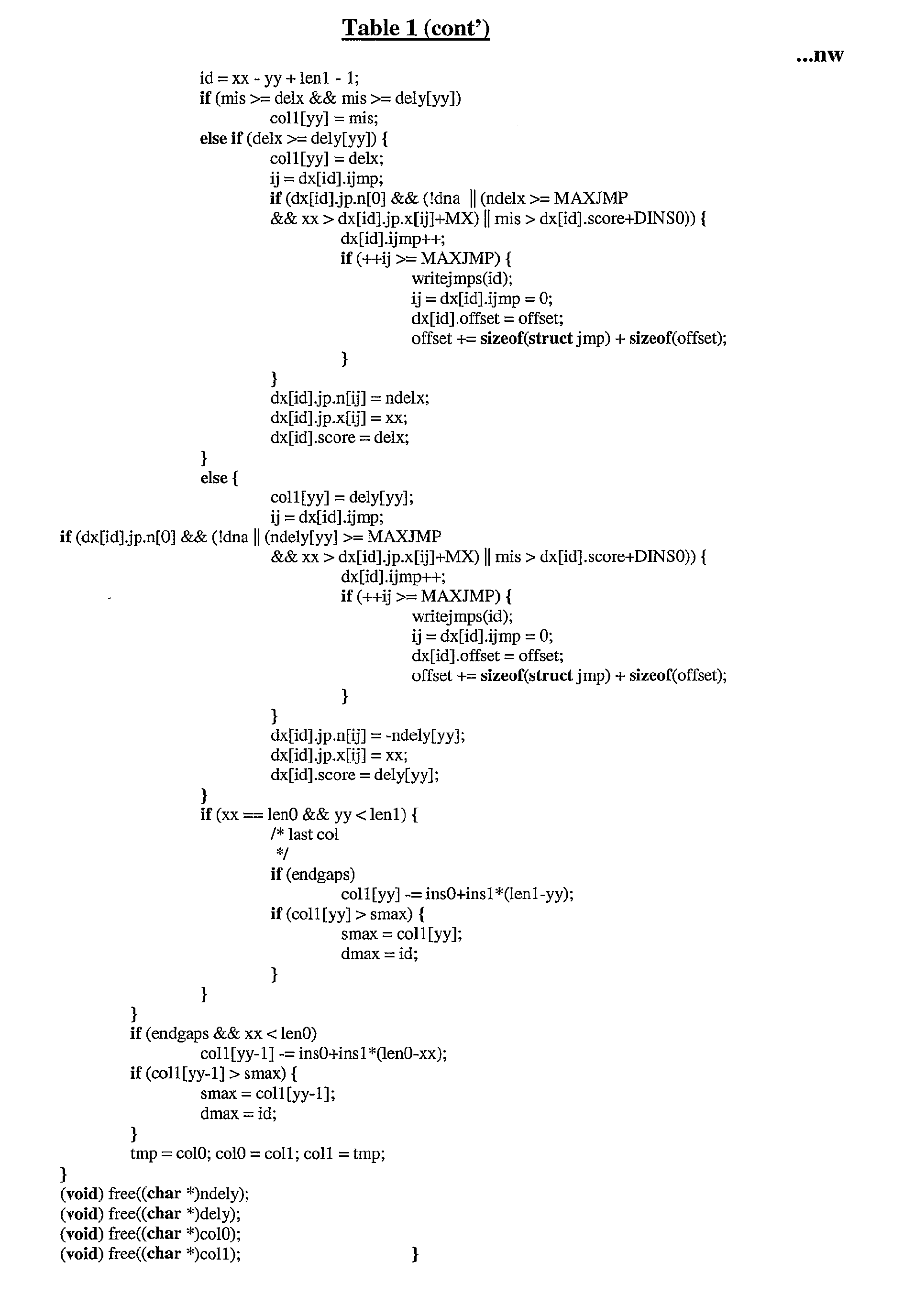

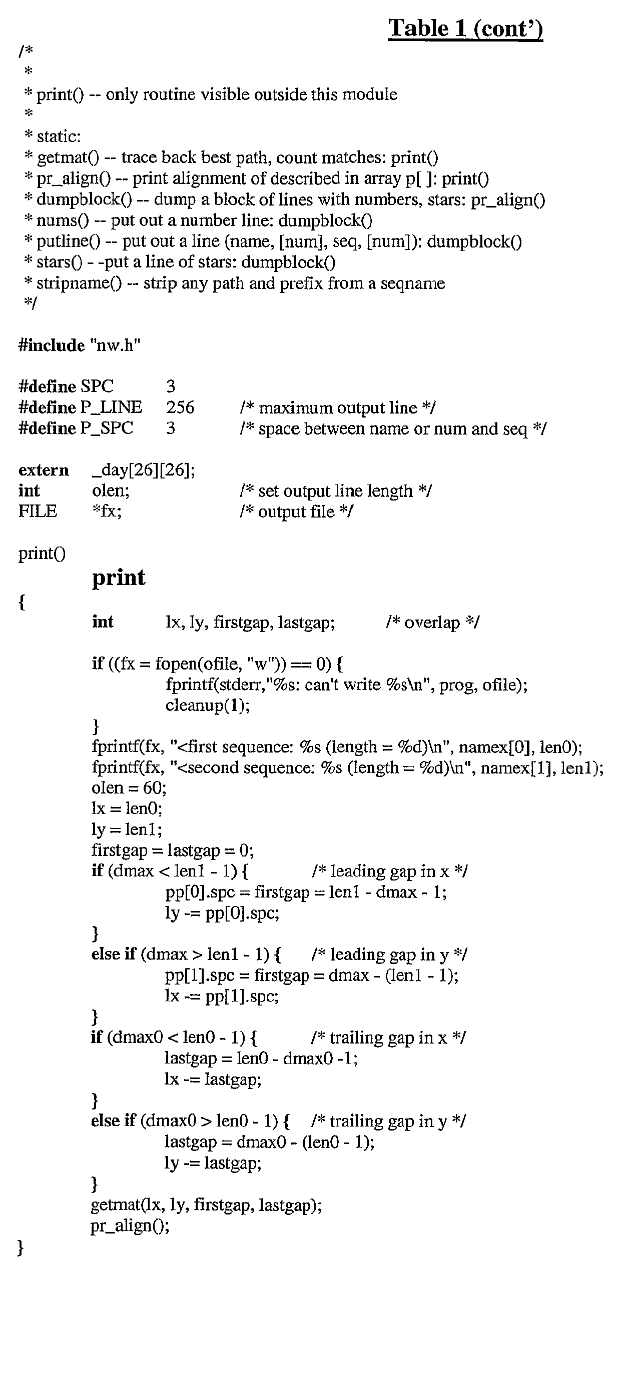

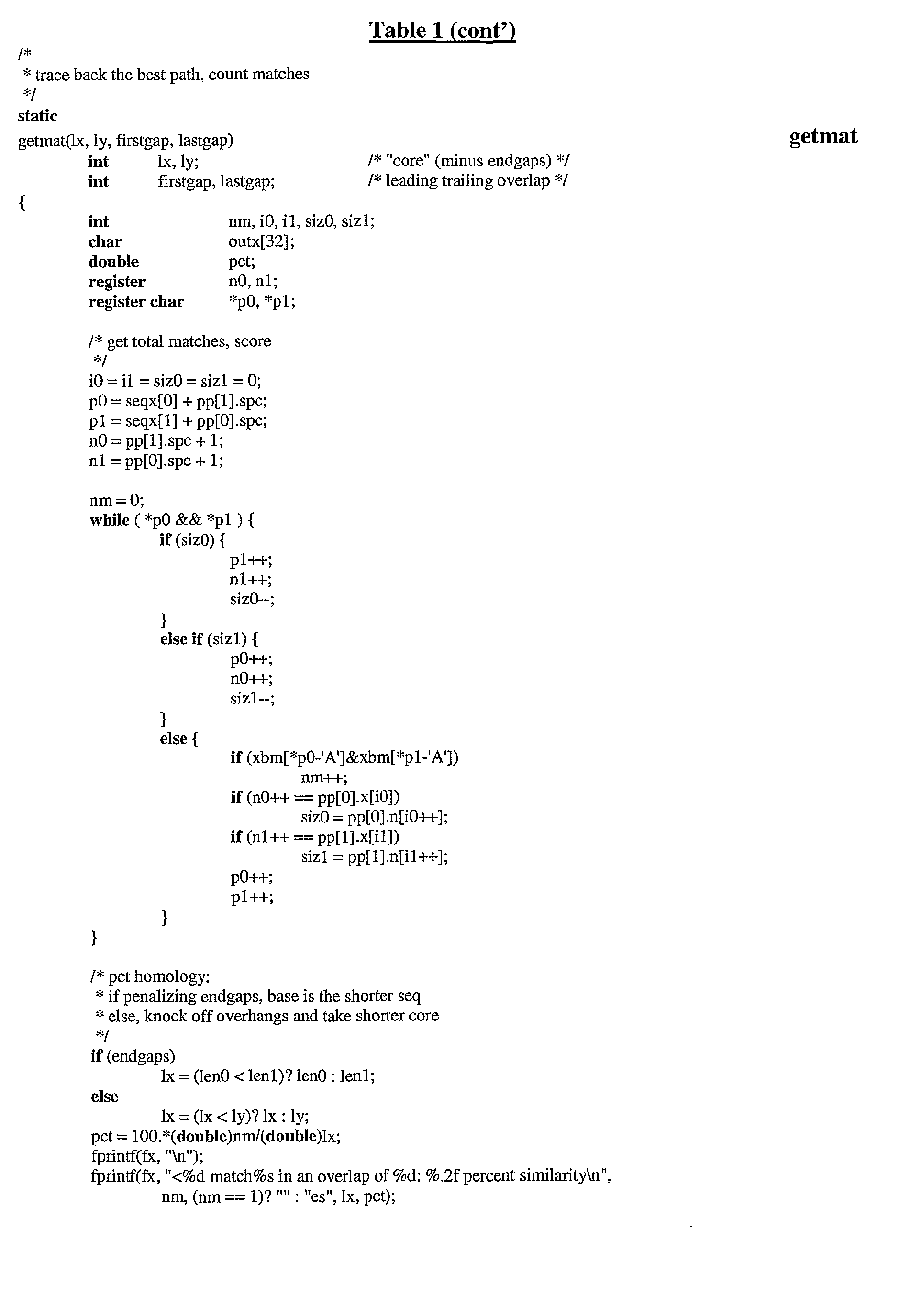

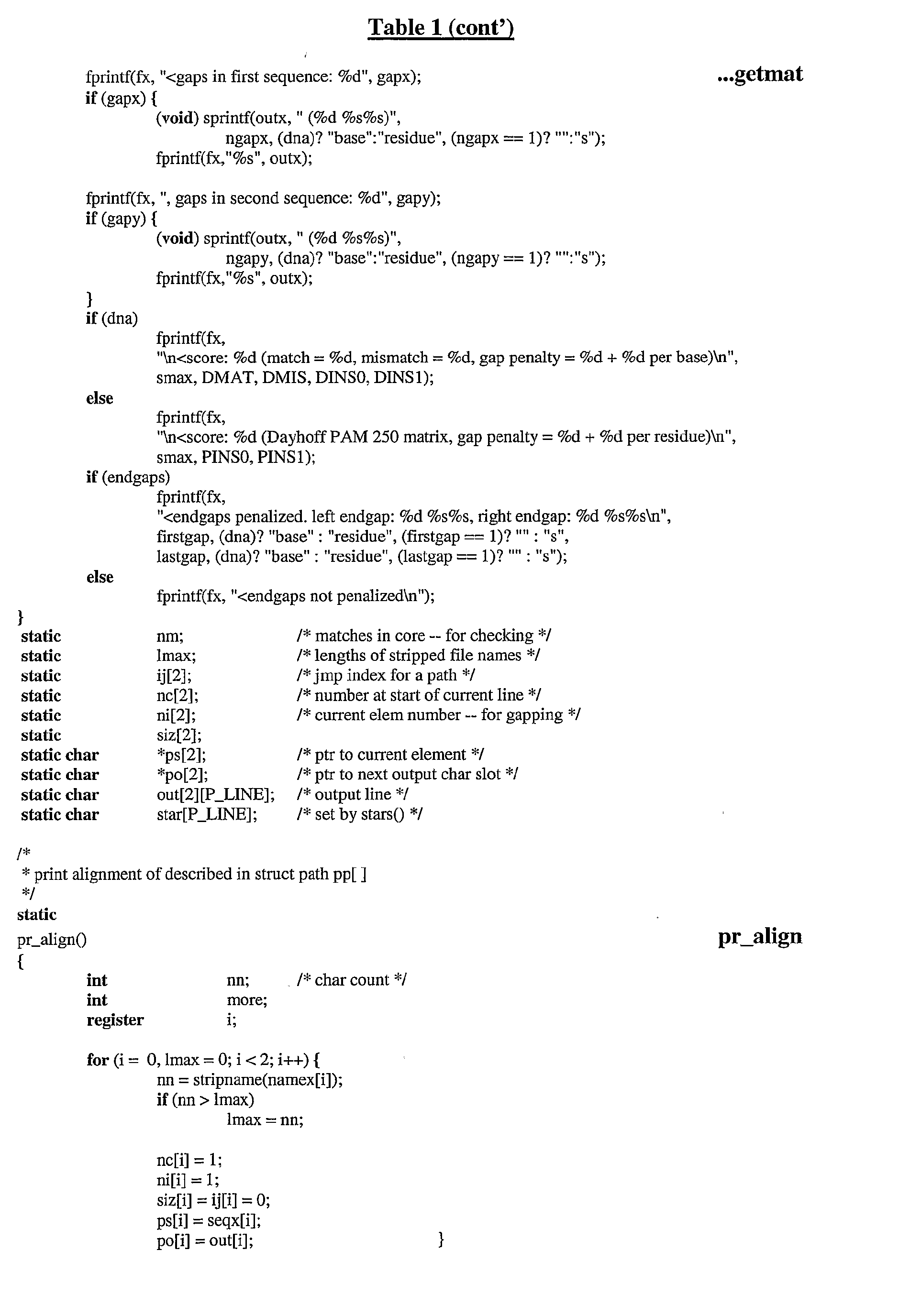

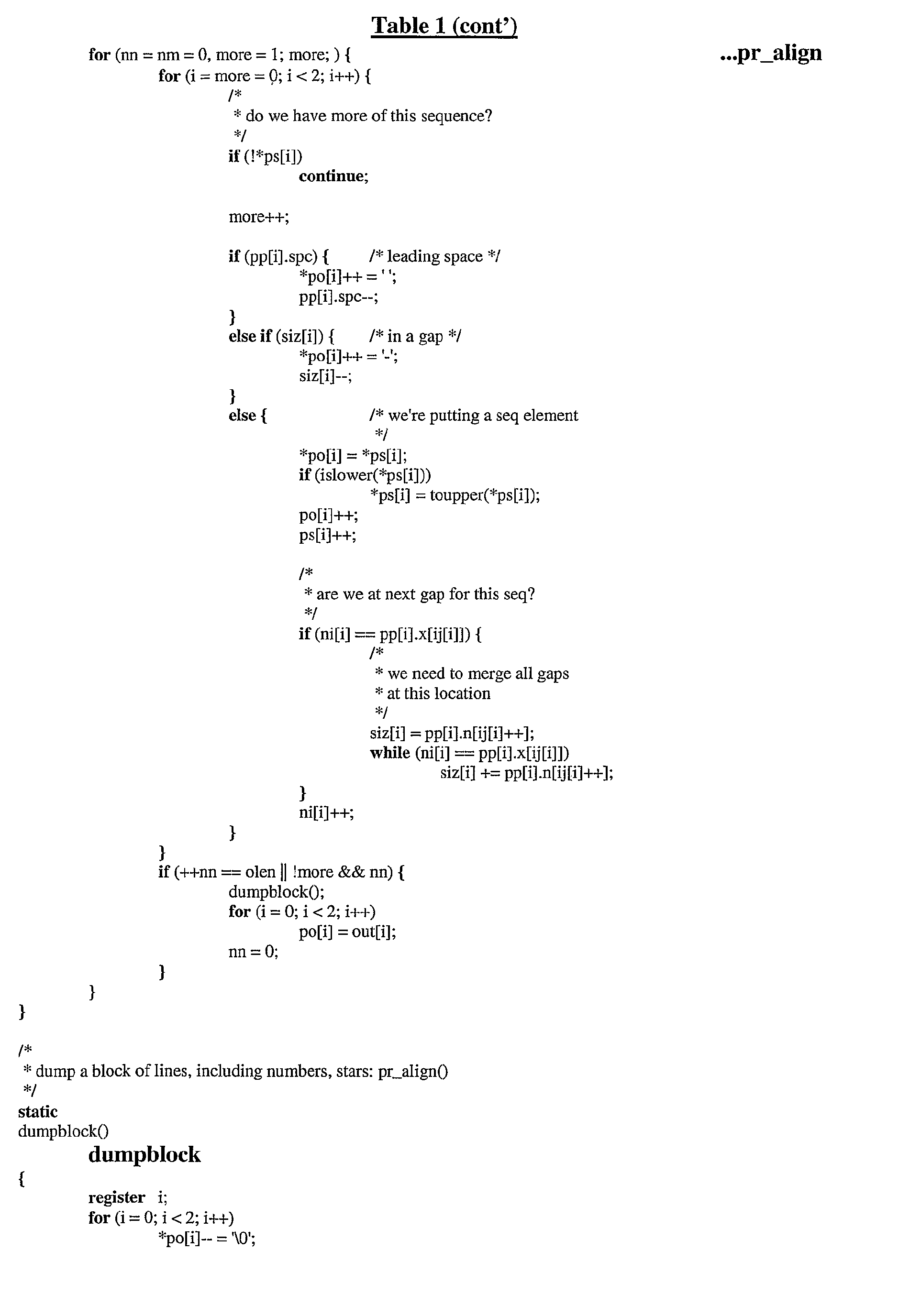

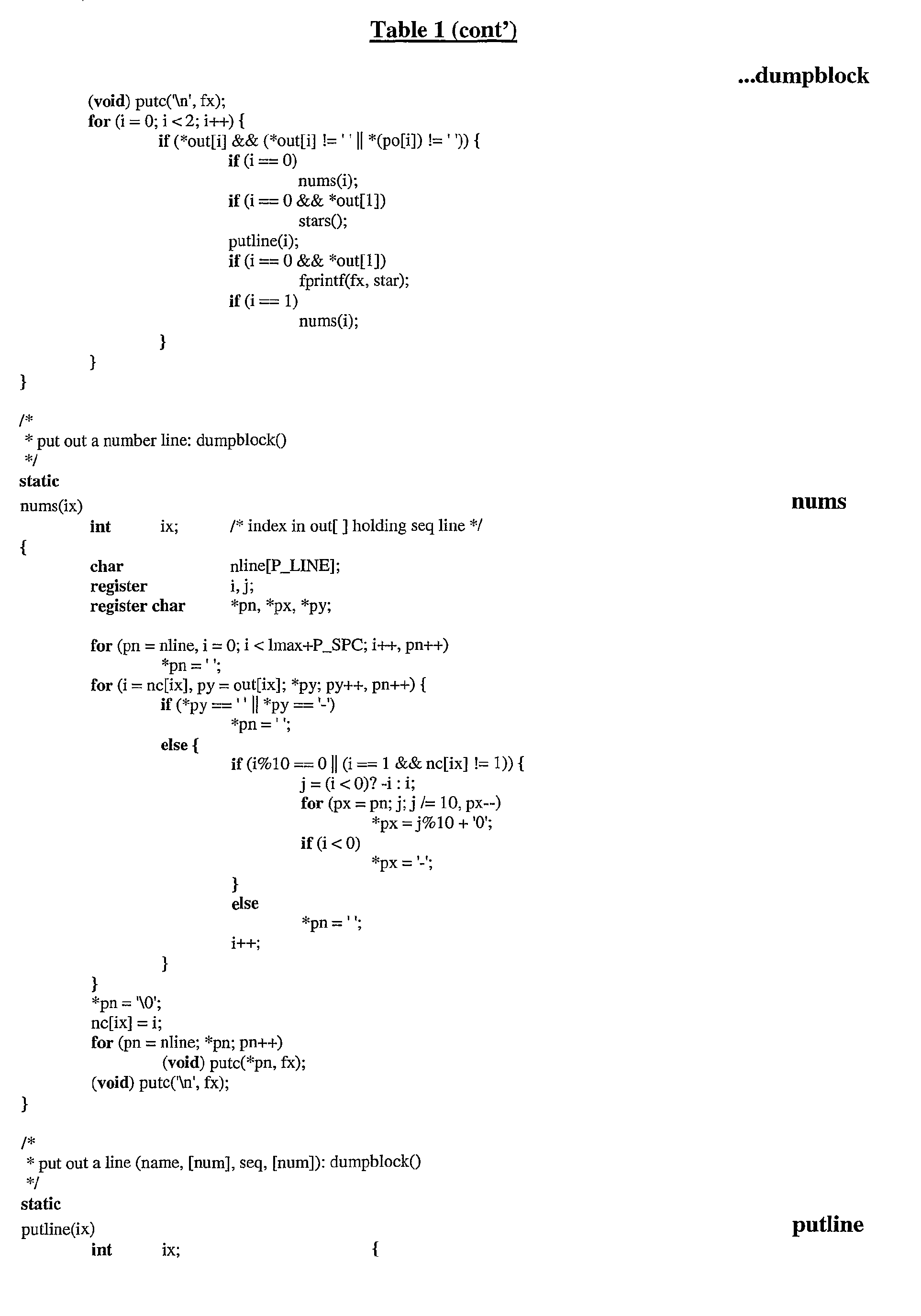

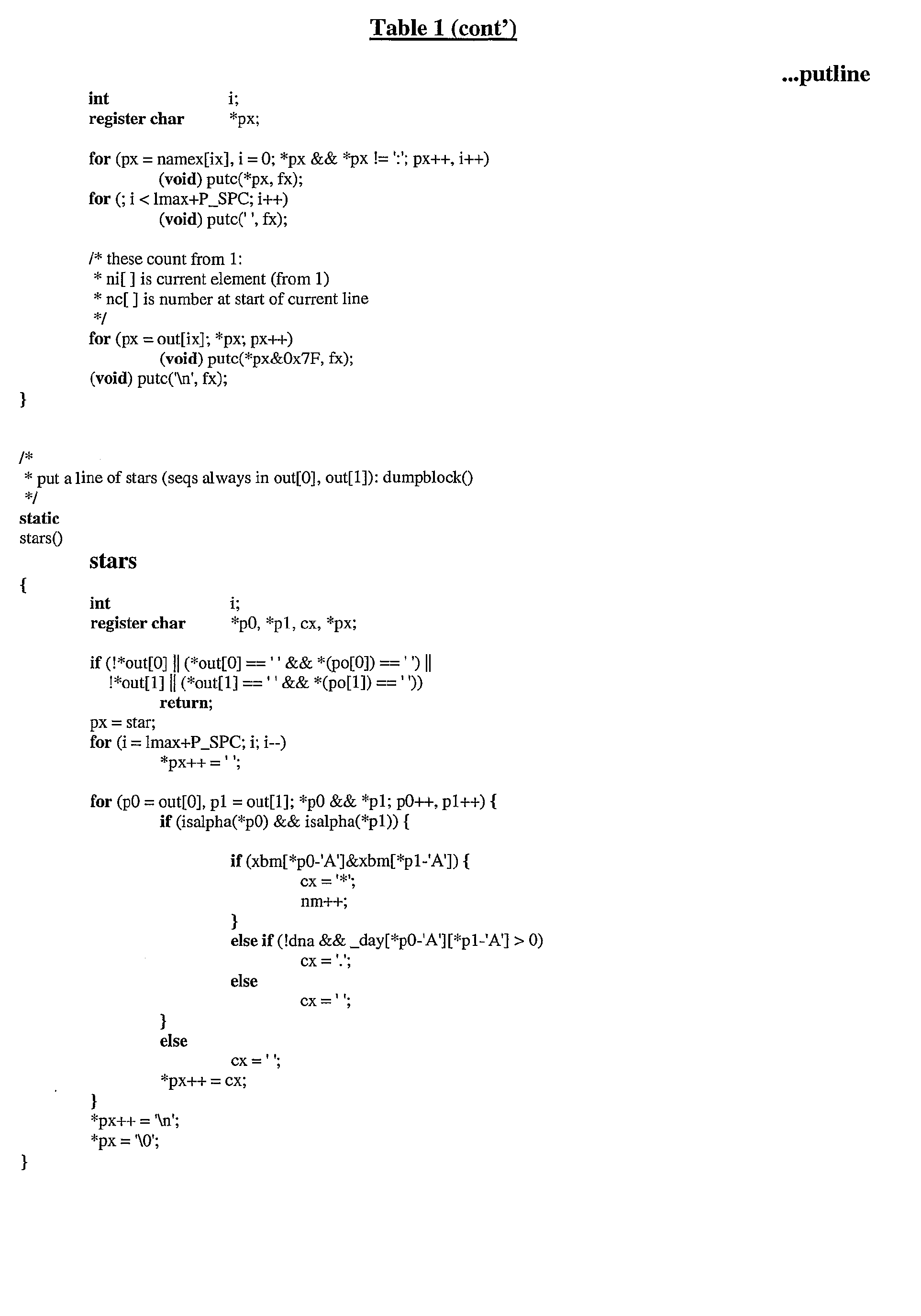

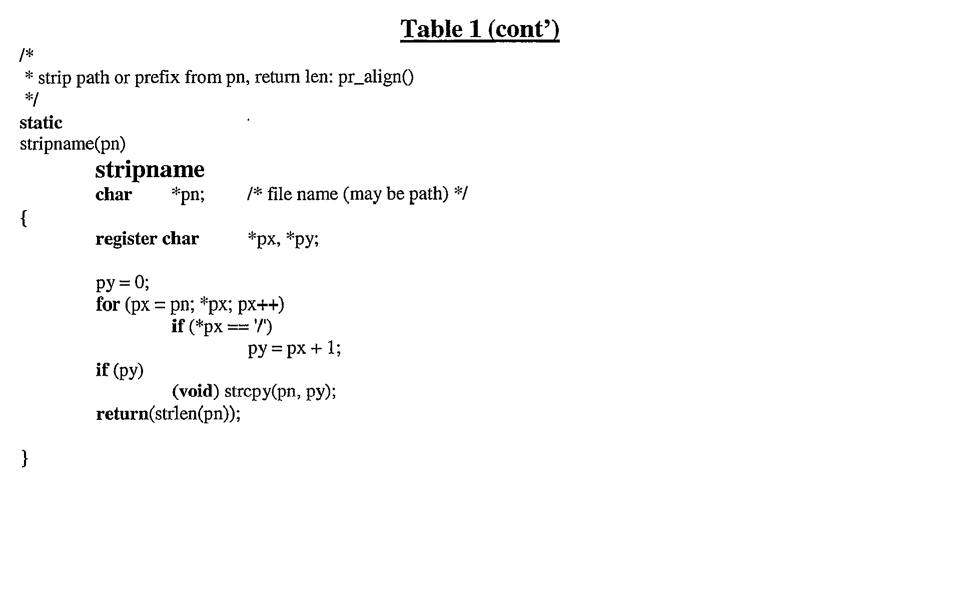

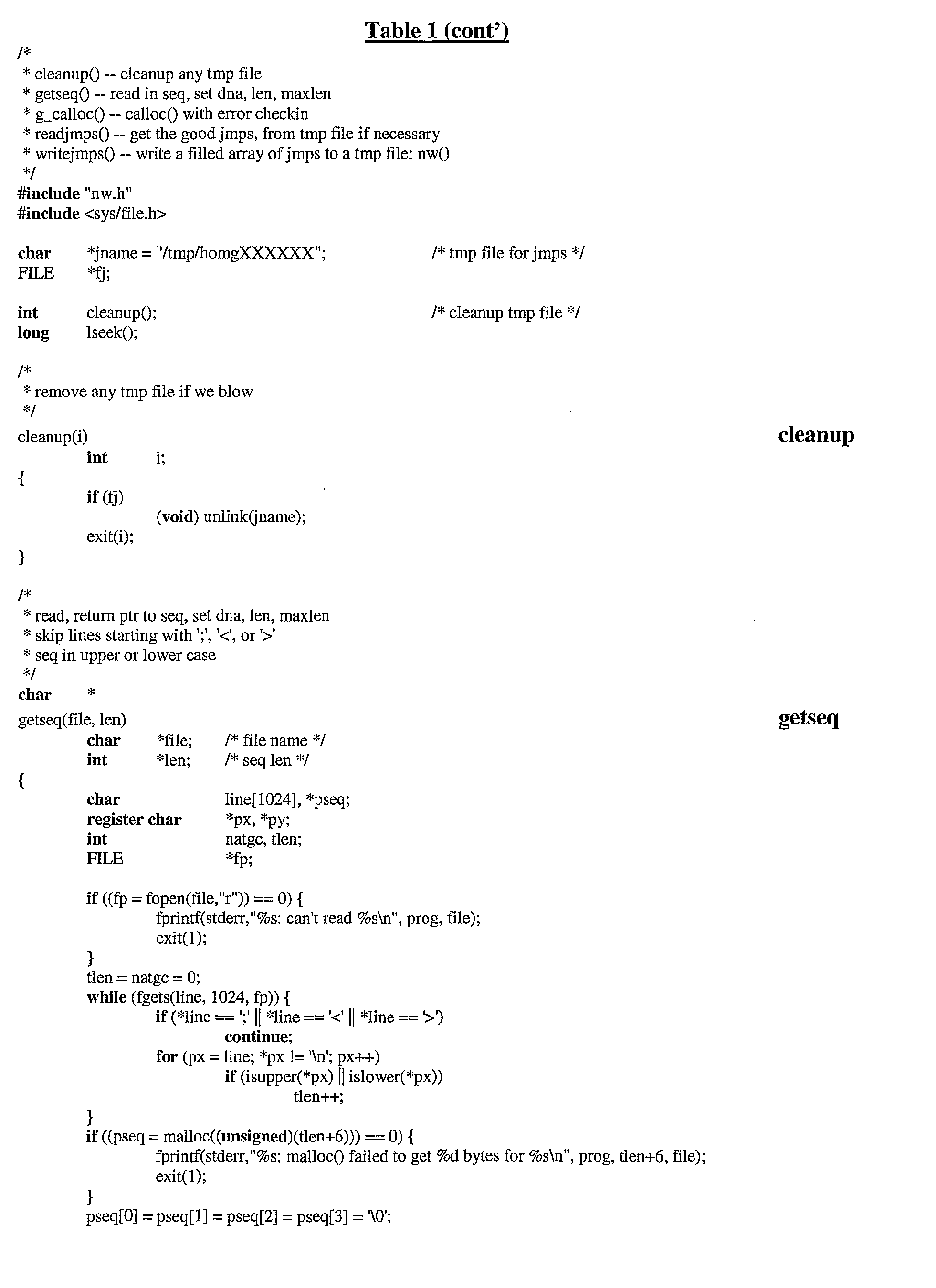

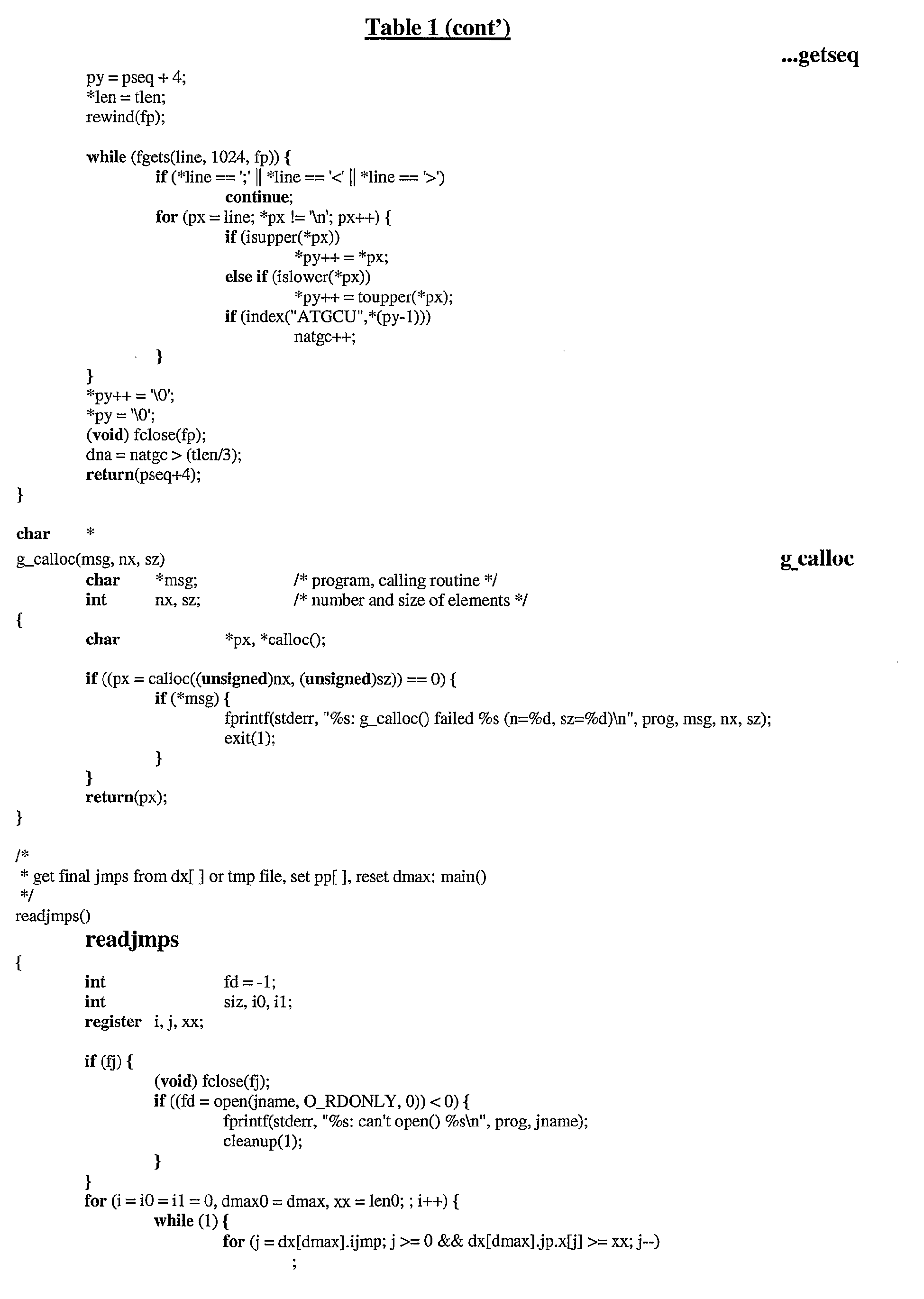

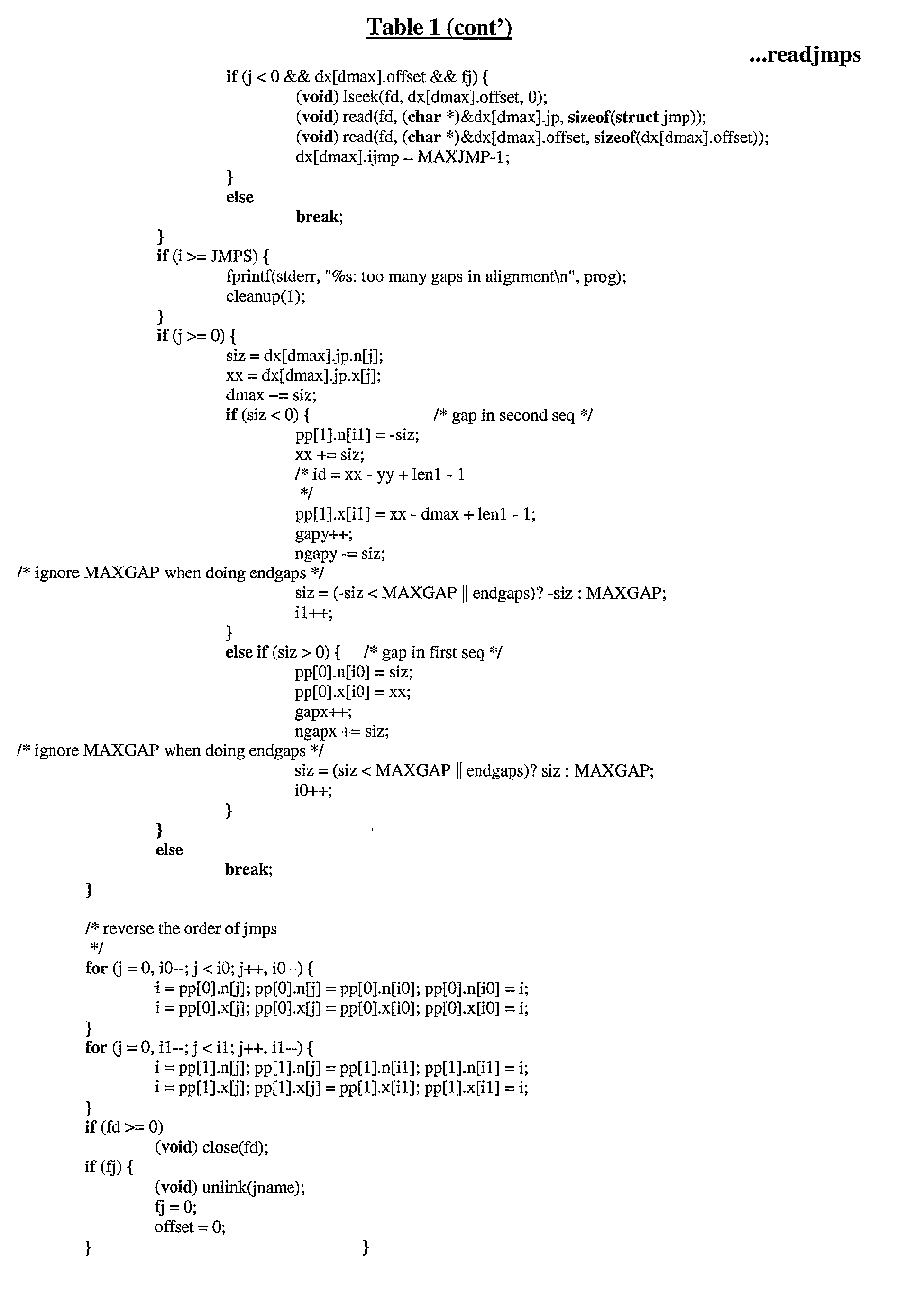

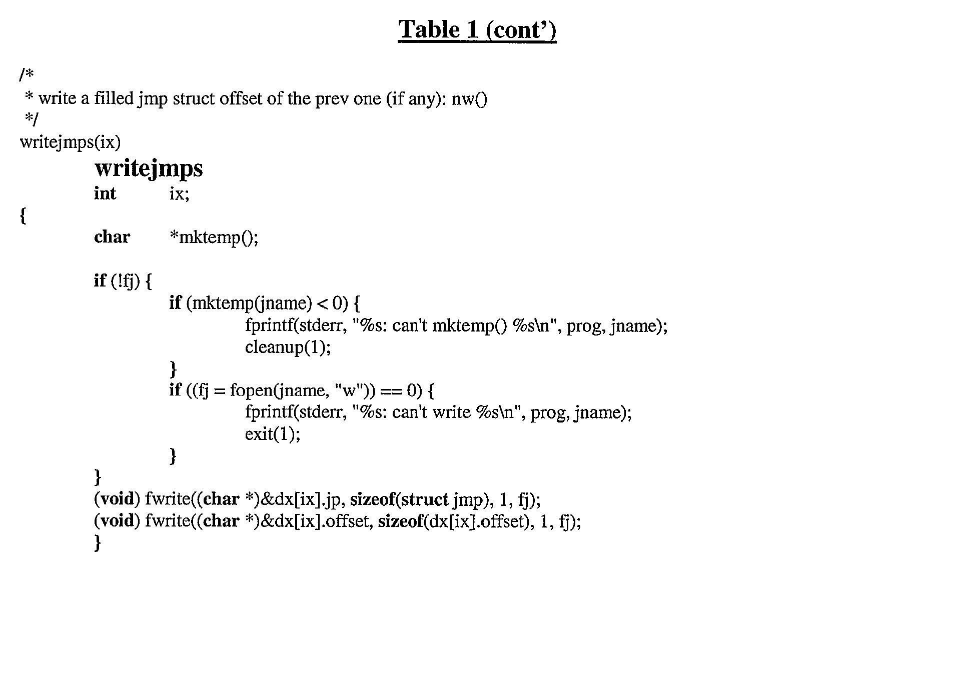

**Table 2**

| | | |
|---|---|---|
| PRO | XXXXXXXXXXXXXXX | (Length = 15 amino acids) |
| Comparison Protein | XXXXXYYYYYYY | (Length = 12 amino acids) |
| % amino acid sequence identity = | | |

(the number of identically matching amino acid residues between the two polypeptide sequences as determined by ALIGN-2) divided by (the total number of amino acid residues of the PRO polypeptide) = 5 divided by 15 = 33.3%

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = | | |

(the number of identically matching amino acid residues between the two polypeptide sequences as determined by ALIGN-2) divided by (the total number of amino acid residues of the PRO polypeptide) = 5 divided by 10 = 50%

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |
| % nucleic acid sequence identity = | | |

(the number of identically matching nucleotides between the two nucleic acid sequences as determined by ALIGN-2) divided by (the total number of nucleotides of the PRO-DNA nucleic acid sequence) = 6 divided by 14 = 42.9%

**Table 5**

| | | |
|---|---|---|
| PRO-DNA | NNNNNNNNNNNN | (Length = 12 nucleotides) |
| Comparison DNA | NNNNLLLW | (Length = 9 nucleotides) |
| % nucleic acid sequence identity = | | |

(the number of identically matching nucleotides between the two nucleic acid sequences as determined by ALIGN-2) divided by (the total number of nucleotides of the PRO-DNA nucleic acid sequence) = 4 divided by 12 = 33.3%

### II. Compositions and Methods of the Invention

### A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been disclosed. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

### B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; | |
| | ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

### 1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan^{r}; E. coli* W3110 strain 37D6, which has the complete genotype *tonA ptr3 phoA E15 (argF-lac)169 degP ompT rbs7 ilvG kan^{r}*; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwannioniyces occidentalis* (EP 394,538 published 31 October 1990); and filamentous fungi such as, e.g., *Neurospora, Perzicillium, Tolypocladium* (WO 91/00357 published 10 January 1991), and *Aspergillus* hosts such as A. *nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A*. *niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, 1pp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C*. *albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

### E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

### F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g*., US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

### G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate NK cell proliferation or inflammatory cell infiltration. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g*., Small et al., Mol. Cell. Biol. 5: 642-648 [1985]).

The use of an agonist stimulating compound has also been validated experimentally. Activation of 4-1BB by treatment with an agonist anti-4-1BB antibody enhances eradication of tumors. Hellstrom, I. and Hellstrom, K. E., Crit. Rev. Immunol. (1998) 18:1. Immunoadjuvant therapy for treatment of tumors, described in more detail below, is another example of the use of the stimulating compounds of the invention.

Alternatively, an immune stimulating or enhancing effect can also be achieved by administration of a PRO which has vascular permeability enhancing properties. Enhanced vascular permeability would be beneficial to disorders which can be attenuated by local infiltration of immune cells (*e.g*., NK cells, monocytes/macrophages, eosinophils, PMNs) and inflammation.

On the other hand, PRO polypeptides, as well as other compounds of the invention, which are direct inhibitors of NK cell proliferation/activation, lymphokine secretion, and/or vascular permeability can be directly used to suppress the immune response. These compounds are useful to reduce the degree of the immune response and to treat immune related diseases characterized by a hyperactive, superoptimal, or autoimmune response. The use of compound which suppress vascular permeability would be expected to reduce inflammation. Such uses would be beneficial in treating conditions associated with excessive inflammation.

Alternatively, compounds, *e.g*., antibodies, which bind to stimulating PRO polypeptides and block the stimulating effect of these molecules produce a net inhibitory effect and can be used to suppress the NK cell mediated immune response by inhibiting NK cell proliferation/activation and/or lymphokine secretion. Blocking the stimulating effect of the polypeptides suppresses the immune response of the mammal.

### H. Animal Models

The results of the cell based in vitro assays can be further verified using *in vivo* animal models and assays for NK cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, *e.g*., murine models. Such models can be generated by introducing cells into syngeneic mice using standard techniques, *e.g*., subcutaneous injection, tail vein injection, spleen implantation, intraperitoneal implantation, implantation under the renal capsule, *etc.*

Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing NK cell reactivity against MHC antigens and minor transplant antigens. A suitable procedure is described in detail in Current Protocols in Immunology, above, unit 4.3.

Contact hypersensitivity is a simple delayed type hypersensitivity *in vivo* assay of cell mediated immune function. In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19 (1): 37-44 (1998)

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g*., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g*., Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g*., head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the NK cell proliferation, stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see *e.g.,* Li et al., Cell, 69:915 (1992)]. The selected cells are then injected into a blastocyst of an animal (e.g., a mouse or rat) to form aggregation chimeras [see *e.g.,* Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### I. ImmunoAdjuvant Therapy

In one embodiment, the immunostimulating compounds of the invention can be used in immunoadjuvant therapy for the treatment of tumors (cancer). It is now well established that NK cells recognize human tumor specific antigens. One group of tumor antigens, encoded by the MAGE, BAGE and GAGE families of genes, are silent in all adult normal tissues , but are expressed in significant amounts in tumors, such as melanomas, lung tumors, head and neck tumors, and bladder carcinomas. DeSmet, C. et al., (1996) Proc. Natl. Acad. Sci. USA, 93:7149. It has been shown that stimulation of immune cells induces tumor regression and an antitumor response both *in vitro* and *in vivo.* Melero, I. et al., Nature Medicine (1997) 3:682; Kwon, E. D. et al., Proc. Natl. Acad. Sci. USA (1997) 94: 8099; Lynch, D. H. et al, Nature Medicine (1997) 3:625; Finn, O. J. and Lotze, M. T., J. Immunol. (1998) 21:114. The stimulatory compounds of the invention can be administered as adjuvants, alone or together with a growth regulating agent, cytotoxic agent or chemotherapeutic agent, to stimulate NK cell proliferation/activation and an antitumor response to tumor antigens. The growth regulating, cytotoxic, or chemotherapeutic agent may be administered in conventional amounts using known administration regimes. Immunostimulating activity by the compounds of the invention allows reduced amounts of the growth regulating, cytotoxic, or chemotherapeutic agents thereby potentially lowering the toxicity to the patient.

### J. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g*., on a microtiter plate, by covalent or non-covalent attachments. Non-covalent attachment generally is accomplished by coating the solid surface with a solution of the polypeptide and drying. Alternatively, an immobilized antibody, *e.g*., a monoclonal antibody, specific for the polypeptide to be immobilized can be used to anchor it to a solid surface. The assay is performed by adding the non-immobilized component, which may be labeled by a detectable label, to the immobilized component, *e.g*., the coated surface containing the anchored component. When the reaction is complete, the non-reacted components are removed, *e.g*., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, coimmunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.
In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

### K. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc.* that inhibit or stimulate immune function, for example, NK cell proliferation/activation, lymphokine release, or immune cell infiltration.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g*., between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g*., Rossi, Current Biology 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, *e.g*., PCT publication No. WO 97/33551, *supra.*

These molecules can be identified by any or any combination of the screening assays discussed above and/or by any other screening techniques well known for those skilled in the art.

### L. Anti-PRO Antibodies

The present invention further provides anti-PRO antibodies. Exemplary antibodies include polyclonal, monoclonal, humanized, bispecific, and heteroconjugate antibodies.

### 1. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E*. *coli* and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from *E*. *coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994). Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, e.g., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e.g*., an enzymatically active toxin of bacterial, fungal, plant, or animal origin, or fragments thereof), or a radioactive isotope (*i.e*., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca. americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g*., avidin) that is conjugated to a cytotoxic agent (*e.g.,* a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### M. Pharmaceutical Compositions

The active PRO molecules of the invention (*e.g*., PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN^{™}, PLURONICS^{™} or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.*, Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, *e.g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### N. Methods of Treatment

It is contemplated that the polypeptides, antibodies and other active compounds of the present invention may be used to treat various immune related diseases and conditions, such as NK cell diseases, including those characterized by infiltration of inflammatory cells into a tissue, stimulation of NK cells, inhibition of NK cells, increased or decreased vascular permeability or the inhibition thereof.

Exemplary conditions or disorders to be treated with the polypeptides, antibodies and other compounds of the invention, include, but are not limited to systemic lupus erythematosis, rheumatoid arthritis, juvenile chronic arthritis, osteoarthritis, spondyloartluopathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, autoantibodies directed against self IgG, with the resultant formation of immune complexes that attain high levels in joint fluid and blood. These complexes in the joint may induce the marked infiltrate of lymphocytes and NK cells into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rheumatoid nodules. As described above, Natural Killer cells are not believed to be involved in the early events of RA, but a study by Dalbeth et al., have found CD56 + NK cells in the synovial fluid of inflamed joints and these cells are believed to respond to IL-12 and IL-15 secreted by macrophages also found in synovial fluid from the inflamed joints (Dalbeth et al., 2002 Arthritis Rheum 46(7): 1763-72).

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. As well as T cells, NK cells are proposed to play a role in the progression of scleroderma in patients where the disease has progressed late into the disease course, and in those patients who did not express anti-Scl 70 antibodies (Holcombe et al., 1995 Ann Rheum Dis 54(1): 69-72). Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase). NK cell activity was specifically studied in patients with Graves' disease and Hashimoto's thyroiditis, and a significant reduction in NK cell activity was found in both patient populations (Wenzel et al., 1998 Thyroid 8(11):1019-1022).

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Psoriasis is a T lymphocyte-mediated inflammatory disease. Lesions contain infiltrates of T lymphocytes, macrophages and antigen processing cells, and some neutrophils.

Other diseases in which intervention of the immune and/or inflammatory response have benefit are infectious disease including but not limited to viral infection (including but not limited to AIDS, hepatitis A, B, C, D, E and herpes) bacterial infection, fungal infections, and protozoal and parasitic infections. Molecules (or derivatives/agonists) which stimulate the immune reaction can be utilized therapeutically to enhance the immune response to infectious agents), diseases of immunodeficiency (molecules/derivatives/agonists) which stimulate the immune reaction can be utilized therapeutically to enhance the immune response for conditions of inherited, acquired, infectious induced (as in HIV infection), or iatrogenic (*i.e.,* as from chemotherapy) immunodeficiency, and neoplasia.

It has been demonstrated that some human cancer patients develop an antibody and/or NK cell response to antigens on neoplastic cells. It has also been shown in animal models of neoplasia that enhancement of the immune response can result in rejection or regression of that particular neoplasm. Molecules that enhance the NK cell response have utility *in vivo* in enhancing the immune response against neoplasia. Molecules which enhance the NK cell proliferative response (or small molecule agonists or antibodies that affected the same receptor in an agonistic fashion) can be used therapeutically to treat cancer. Molecules that inhibit the NK cell response also function *in vivo* during neoplasia to suppress the immune response to a neoplasm; such molecules can either be expressed by the neoplastic cells themselves or their expression can be induced by the neoplasm in other cells. Antagonism of such inhibitory molecules (either with antibody, small molecule antagonists or other means) enhances immune-mediated tumor rejection.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g*., polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (*e.g.,* 0.1-20 mg/kg) of polypeptide or antibody is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

### O. Articles of Manufacture

In another embodiment of the invention, an article of manufacture containing materials (*e.g*., comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### P. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, *e.g*., fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Microarray analysis of NK cells.

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (in this instance, activated NK cells) sample is greater than hybridization signal of a probe from a control (in this instance, resting NK cells) sample, the gene or genes expressed in the test tissue are identified. The implication of this result is that an overexpressed protein in a test tissue is useful not only as a diagnostic marker for the presence of the disease condition, but also as a therapeutic target for treatment of the disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

For this experiment, peripheral blood NK cells were isolated from leukopacks by negative selection using the NK cell isolation kit with the Miltenyi MACS™ magnetic cell sorting system (Miltenyi Biotec). Cell purity was confirmed by staining with PE anti-CD56 for FACS analysis. Purity of cell preps ranged from 89% to 96%. Cell culture conditions were as follows: Set up in-vitro cultures in 6 well plates - 5 ml cultures/well using RPMI 1640, 10%heat inactivated FBS, 100 units/mL of Penicillin, 100 mg/mL of streptomycin, 2 mM L-glutamine, and 5.5 x 10-5 Beta-mercaptoethanol. The cells were cultured for 16 hours in the base media described above, but with the addition of IL-12 (10nM), IL-15(10nM) or JAM-IT(10nM). An untreated control sample was also cultured for 16 hours (16 Hour Timepoint). Activation of NK cells was monitored by FACS for cell surface expression of CD56 and CD69. Uncultured, untreated CD56(+) cells, for use as a control (Time 0). RNA was isolated using the Qiagen Midi™ preps as per the instructions in the manual with the addition of an on-column DNAse I digestion after the first RW1 wash step. RNA was eluted in RNAse free water and subsequently concentrated by ethanol precipitation. Precipitated RNA was taken up in nuclease free water to a final minimum concentration of 0.5 micrograms per microliter.

The isolated mRNA was labeled and hybridized to Affimax™ (Affymetrix Inc. Santa Clara, CA) microarray chips and proprietary Genentech microarrays. The cells harvested at Time 0 timepoint, and the 16 Hour timepoint were subjected to the same analysis. Genes were compared whose expression was upregulated at the 16 Hour Timepoint as compared to untreated 16 Hour Timepoint and uncultured, untreated Time 0 timepoints.

Below are the results of these experiments, demonstrating that various PRO polypeptides of the present invention are differentially expressed in activated NK cells after 16 hours in culture with IL-12, IL-15 or JAM2 as compared to normal resting NK cells cultured for 16 hours or resting NK cells harvested at Time 0. Specifically, Figure 438A-B, Figure 511, Figure 687, Figure 697, Figure 703, Figure 1057, Figure 1084, Figure 1126 and Figure 1228 identify sequences that are highly overexpressed in activated NK cells as compared to normal resting NK cells.
As described above, these data demonstrate that the PRO polypeptides of the present invention are useful not only as diagnostic markers for the presence of one or more immune disorders, but also serve as therapeutic targets for the treatment of those immune disorders.

Figures 1-1477 (SEQ ID NOS: 1-1477) show the nucleic acids of the invention and their encoded PRO polypeptides.

### EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM 88 sodium phosphate, pH 6.8, 2x Denhardt's solution, and 10% dextran sulfate at 42°C for 20 hours. Washing of the filters is performed in an aqueous solution of 0.1x SSC and 0.1 % SDS at 42°C.

DNAs having a desired sequence identity with the DNA encoding full-length native sequence PRO can then be identified using standard techniques known in the art.

### EXAMPLE 3: Expression of PRO in E. coli

This example illustrates preparation of an unglycosylated form of PRO by recombinant expression in *E*. *coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E*. *coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E*. *coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E*. *coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E*. *coli* host based on strain 52 (W3110 fuhA(tonA) lon galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate·2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

### EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 [µg pRK5-PRO DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression. The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Quiagen), Dosper^{®} or Fugene^{®} (Boehringer Mannheim). The cells are grown as described in Lucas et al., supra. Approximately 3 x 10⁻⁷ cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

### EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²+-NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (*e.g*., high ionic strength buffers in the presence of detergent). Then, the column is eluted under conditions that disrupt antibody/PRO polypeptide binding (*e.g*., a low pH buffer such as approximately pH 2-3, or a high concentration of a chaotrope such as urea or thiocyanate ion), and PRO polypeptide is collected.

### EXAMPLE 9: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide%ell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 10: Rational Drug Design

The goal of rational drug design is to produce structural analogs of biologically active polypeptide of interest (*i.e.,* a PRO polypeptide) or of small molecules with which they interact, *e.g.,* agonists, antagonists, or inhibitors. Any of these examples can be used to fashion drugs which are more active or stable forms of the PRO polypeptide or which enhance or interfere with the function of the PRO polypeptide *in vivo (c.f.,* Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

The following numbered paragraphs (paras.) contain further statements of various aspects of the present invention:-
1. Isolated nucleic acid having at least 80% nucleic acid sequence identity to a nucleotide sequence identity to:
   (a) the nucleotide sequence shown as any one of the Figures 1-1477 (SEQ ID NOS: 1-1477); or
   (b) the nucleotide sequence encoding the polypeptide shown as any one of the Figures 1-1477 (SEQ ID NOS: 1-1477).
2. A vector comprising the nucleic acid of Para. 1.
3. The vector of Para. 2 operably linked to control sequences recognized by a host cell transformed with the vector.
4. A host cell comprising the vector of Para. 2.
5. The host cell of Para. 4, wherein said cell is a CHO cell, an *E. coli* cell or a yeast cell.
6. A process for producing a PRO polypeptide comprising culturing the host cell of Para. 5 under conditions suitable for expression of said PRO polypeptide and recovering said PRO polypeptide from the cell culture.
7. An isolated polypeptide having at least 80% amino acid sequence identity to:
   (a) a polypeptide shown as any one of Figures 1-1477 (SEQ ID NOS: 1-1477); or
   (b) a polypeptide encoded by the full length coding region of the nucleotide sequence shown as any one of Figures 1-1477 (SEQ ID NOS: 1-1477).
8. A chimeric molecule comprising a polypeptide according to Para. 7 fused to a heterologous amino acid sequence.
9. The chimeric molecule of Para. 8, wherein said heterologous amino acid sequence is an epitope tag sequence or an Fc region of an immunoglobulin.
10. An antibody which specifically binds to a polypeptide according to Para. 7.
11. The antibody of Para. 10, wherein said antibody is a monoclonal antibody, a humanized antibody or a single-chain antibody.
12. A composition of matter comprising (a) a polypeptide of Para. 7, (b) an agonist of said polypeptide, (c) an antagonist of said polypeptide, or (d) an antibody that binds to said polypeptide, in combination with a carrier.
13. The composition of matter of Para. 12, wherein said carrier is a pharmaceutically acceptable carrier.
14. The composition of matter of Para. 13 comprising a therapeutically effective amount of (a), (b), (c) or (d).
15. An article of manufacture, comprising:
   a container;
   a label on said container; and
   a composition of matter comprising (a) a polypeptide of Para. 7, (b) an agonist of said polypeptide, (c) an antagonist of said polypeptide, or (d) an antibody that binds to said polypeptide, contained within said container, wherein label on said container indicates that said composition of matter can be used for treating an immune related disease.
16. A method of treating an immune related disorder in a mammal in need thereof comprising administering to said mammal a therapeutically effective amount of (a) a polypeptide of Para. 7, (b) an agonist of said polypeptide, (c) an antagonist of said polypeptide, or (d) an antibody that binds to said polypeptide.
17. The method of Para. 16, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.
18. A method for determining the presence of a PRO polypeptide of the invention as described in Figures 1-1477 (SEQ ID NOS: 1-1477), in a sample suspected of containing said polypeptide, said method comprising exposing said sample to an anti-PRO antibody, where the and determining binding of said antibody to a component of said sample.
19. A method of diagnosing an immune related disease in a mammal, said method comprising detecting the level of expression of a gene encoding a PRO polypeptide of the invention as described in Figures 1-1477 (SEQ ID NOS: 1-1477), (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.
20. A method of diagnosing an immune related disease in a mammal, said method comprising (a) contacting a PRO polypeptide of the invention as described in Figures 1-1477 (SEQ ID NOS: 1-1477), anti-PRO antibody with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.
21. A method of identifying a compound that inhibits the activity of a PRO polypeptide of the invention as described in Figures 1-1477 (SEQ ID NOS: 1-1477), said method comprising contacting cells which normally respond to said polypeptide with (a) said polypeptide and (b) a candidate compound, and determining the lack responsiveness by said cell to (a).
22. A method of identifying a compound that inhibits the expression of a gene encoding a PRO polypeptide of the invention as described in Figures 1-1477 (SEQ ID NOS: 1-1477), said method comprising contacting cells which normally express said polypeptide with a candidate compound, and determining the lack of expression said gene.
23. The method of Para. 22, wherein said candidate compound is an antisense nucleic acid.
24. A method of identifying a compound that mimics the activity of a PRO polypeptide of the invention as described in any one of Figures 1-1477 (SEQ ID NOS: 1-1477), said method comprising contacting cells which normally respond to said polypeptide with a candidate compound, and determining the responsiveness by said cell to said candidate compound.
25. A method of stimulating the immune response in a mammal, said method comprising administering to said mammal an effective amount of a PRO polypeptide of the invention as described in any one of Figures 1-1477 (SEQ ID NOS: 1-1477), antagonist, wherein said immune response is stimulated.
26. A method of diagnosing an inflammatory immune response in a mammal, said method comprising detecting the level of expression of a gene encoding a PRO polypeptide of the invention as described in any one of Figures 1-1477 (SEQ ID NOS: 1-1477), (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.

## Claims

1. A method of diagnosing an immune related disease or an inflammatory immune response in a mammal, said method comprising:
detecting the level of expression of a gene encoding a PR02398 or PRO51442 polypeptide, wherein the gene (i) has at least 80% nucleic acid sequence identity to the nucleic acid sequence shown as Figure 905 (SEQ ID NO: 905) or Figure 546 (SEQ ID NO: 546), respectively, or (ii) encodes a polypeptide having at least 80% amino acid sequence identity to the amino acid sequence shown as Figure 906 (SEQ ID NO: 906) or Figure 547 (SEQ ID NO: 547), respectively, (a) in a test sample of tissue cells obtained from the mammal, and (b) in a control sample of known normal tissue cells of the same cell type, wherein a differential level of expression of said gene in the test sample as compared to the control sample is indicative of the presence of an immune related disease in the mammal from which the test tissue cells were obtained; or
(a) contacting an anti-PRO2398 or anti-PRO51442 antibody to a polypeptide having at least 80% amino acid sequence identity to (i) the polypeptide sequence shown as Figure 906 (SEQ ID NO: 906) or Figure 547 (SEQ ID NO: 547), respectively, or (ii) a polypeptide encoded by the full length coding region of the nucleotide sequence shown as Figure 905 (SEQ ID NO: 905) or Figure 546 (SEQ ID NO: 546), respectively, with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.

2. The method of claim 1, wherein a higher level of expression of said gene in the test sample as compared to the control sample is indicative of the presence of an immune related disease in the mammal from which the test tissue cells were obtained.

3. The method of claim 1 or 2, wherein the gene encoding a PR02398 or PRO51442 polypeptide (i) has at least 90% or at least 95% nucleic acid sequence identity to the nucleic acid sequence shown as Figure 905 (SEQ ID NO: 905) or Figure 546 (SEQ ID NO: 546), respectively, or (ii) encodes a polypeptide having at least 90% or at least 95% amino acid sequence identity to the amino acid sequence shown as Figure 906 (SEQ ID NO: 906) or Figure 547 (SEQ ID NO: 547), respectively.

4. The method of claim 1 or 2, wherein the gene encoding a PR02398 or PRO51442 polypeptide (i) has the nucleic acid sequence shown as Figure 905 (SEQ ID NO: 905) or Figure 546 (SEQ ID NO: 546), respectively, or (ii) encodes a polypeptide having the amino acid sequence shown as Figure 906 (SEQ ID NO: 906) or Figure 547 (SEQ ID NO: 547), respectively.

5. The method of claim 1, wherein the anti-PRO2398 or anti-PRO51442 antibody is to a polypeptide (i) having the sequence shown as Figure 906 (SEQ ID NO: 906) or Figure 547 (SEQ ID NO: 547), respectively, or (ii) encoded by the full length coding region of the nucleotide sequence shown as Figure 905 (SEQ ID NO: 905) or Figure 546 (SEQ ID NO: 546), respectively.

6. The method of any one of claims 1 to 5, wherein the immune related disease is an NK cell related disease.

7. The method of any one of claims 1 to 6, wherein the immune related disease is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.

8. A molecule which is (a) a PR02398 or PRO51442 polypeptide having at least 80% amino acid sequence identity to (i) the polypeptide sequence shown as Figure 906 (SEQ ID NO: 906) or Figure 547 (SEQ ID NO: 547), respectively, or (ii) a polypeptide encoded by the full length coding region of the nucleotide sequence shown as Figure 905 (SEQ ID NO: 905) or Figure 546 (SEQ ID NO: 546), respectively, (b) an agonist of said polypeptide, (c) an antagonist of said polypeptide, or (d) an antibody that binds to said polypeptide, for use in a method of treating an immune related disorder in a mammal in need thereof comprising administering to said mammal a therapeutically effective amount of said molecule.

9. The molecule for use of claim 8, wherein the immune related disorder is an NK cell related disorder.

10. The molecule for use of claim 9, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.

11. A method of identifying a compound that inhibits the activity of a PR02398 or PRO51442 polypeptide having at least 80% amino acid sequence identity to (i) the polypeptide sequence shown as Figure 906 (SEQ ID NO: 906) or Figure 547 (SEQ ID NO: 547), respectively, or (ii) a polypeptide encoded by the full length coding region of the nucleotide sequence shown as Figure 905 (SEQ ID NO: 905) or Figure 546 (SEQ ID NO: 546), respectively, said method comprising contacting cells which normally respond to said polypeptide with (a) said polypeptide and (b) a candidate compound, and determining the lack responsiveness by said cell to (a).

12. A method of identifying a compound that inhibits the expression of a gene encoding a PR02398 or PRO51442 polypeptide, wherein the gene (i) has at least 80% nucleic acid sequence identity to the nucleic acid sequence shown as Figure 905 (SEQ ID NO: 905) or Figure 546 (SEQ ID NO: 546), respectively, or (ii) encodes a polypeptide having at least 80% amino acid sequence identity to the amino acid sequence shown as Figure 906 (SEQ ID NO: 906) or Figure 547 (SEQ ID NO: 547), respectively, said method comprising contacting cells which normally express said polypeptide with a candidate compound, and determining the lack of expression said gene.

13. The method of Claim 12, wherein said candidate compound is an antisense nucleic acid.

14. A method of identifying a compound that mimics the activity of a PR02398 or PRO51442 polypeptide having at least 80% amino acid sequence identity to (i) the polypeptide sequence shown as Figure 906 (SEQ ID NO: 906) or Figure 547 (SEQ ID NO: 547), respectively, or (ii) a polypeptide encoded by the full length coding region of the nucleotide sequence shown as Figure 905 (SEQ ID NO: 905) or Figure 546 (SEQ ID NO: 546), respectively, said method comprising contacting cells which normally respond to said polypeptide with a candidate compound, and determining the responsiveness by said cell to said candidate compound.

15. A PR02398 or PRO51442 polypeptide having at least 80% amino acid sequence identity to (i) the polypeptide sequence shown as Figure 906 (SEQ ID NO: 906) or Figure 547 (SEQ ID NO: 547), respectively, or (ii) a polypeptide encoded by the full length coding region of the nucleotide sequence shown as Figure 905 (SEQ ID NO: 905) or Figure 546 (SEQ ID NO: 546), respectively, or antagonist, for use in a method of stimulating the NK cell response in a mammal comprising administering to said mammal an effective amount of said molecule.
